# EUROPEAN PATENT APPLICATION

(11) **EP 2 095 821 A1**
(43) Date of publication of application: **02.09.2009**
(21) Application number: 08152180.9
(22) Date of filing: 29.02.2008
(51) Int. Cl.: A61K 38/17, A61K 38/10, A61P 35/00

(54) **Anticancer agent**

(71) Applicant: Rheinische Friedrich-Wilhelms-Universität Bonn, 53113 Bonn (DE)
(72) Inventor: Hoch, Michael Karl, Dr., 53913 Swisttal (DE); Kolanus, Waldemar, Dr., 53127 Bonn (DE); Schultze, Waldemar, Dr., 50259 Pulheim-Simmersdorf (DE); Loer, Birgit, 53127 Bonn (DE)
(74) Representative: Althausen, Sonja

(57) **Abstract**

The present invention relates to a pharmaceutical composition for the therapeutic and/or prophylactic treatment of cancer and/or metastasis thereof comprising an amino acid sequence related to a Wech protein and/or a nucleic acid sequence of the gene encoding for the Wech protein.

## Description

The present invention relates to a pharmaceutical composition. More particularly, it concerns the use of new genes and their respective encoded proteins in a pharmaceutical composition for the treatment of cancer.

Cancer is a major world-wide health problem. Although extensive research around the world has led to advances in cancer treatment, progress has been slow and there is no known cure. The control of cancer is still a most important subject on today's medicine, and new cancer therapy and new anti-cancer agents are topics of utmost interest among medical and pharmaceutical researchers.

Known methods of cancer treatment for example use chemotherapeutic anti-cancer agents. However, the use of existing compounds such as alkylating agents making use of cytotoxicity is considerably limited owing to manifest side effects. Moreover, tumor cell resistance to chemotherapeutic agents represents a significant problem in clinical oncology, being one of the main reasons why many of the most prevalent forms of human cancer still resist effective chemotherapeutic intervention, despite certain advances in the field of chemotherapy.

Other known methods in cancer treatment use antibodies. However, antibodies are not free of serious side effects either. Moreover, antigen-negative or antigen-deficient cells can survive and repopulate the tumor or lead to further metastases. Moreover, antibodies are not a treatment of choice for treating solid tumors.

However, modern molecular biological techniques have contributed to our understanding of the genetic aspects of cancer development. So, numerous scientific studies have established that in the development of cancer gene mutations are involved including inactivation of tumor suppressor genes. Tumor-suppressor genes are genes that normally prevent cells from growing out of control. The loss or silencing of one or more tumor-suppressor genes is believed to be an important part of cancer development. Therefore, the administration of tumor suppressor genes is another useful strategy for the prevention and/or treatment of cancer.

However, new therapeutic modalities for the treatment of cancer are urgently needed.

Therefore, an object of the present invention is to provide a novel agent for the treatment of cancer.

This object is met with means according to the independent claims of the present invention. The dependent claims are related to preferred embodiments.

The term "cancer" as used herein refers to or describes the physiological condition, preferably in a mammalian subject, that is typically characterized by abnormal or unregulated cell growth, often being capable of invading adjacent tissues and spreading to distant organs. The term "cancer" as used herein includes carcinomas, germ cell tumors, neoplasms particularly malignant neoplasms or malignant tumors, and pre-malignant conditions.

Cancer is usually classified according to the tissue from which the cancerous cells originate, as well as the normal cell type they most resemble. Examples of cancer include, but are not limited to, the group comprising thyroid cancer, lung cancer, small cell lung cancer (SCLC), liver cancer, cancers of the atrioventricular node, cancers of the skeletal muscle, skin cancer, salivary gland cancer, ovary cancer, upper gastrointestinal cancers preferably selected from the group comprising pancreas cancer, esophagus cancer, and/or stomach cancer, and/or cancers of the nervous system preferably selected from the group comprising cancers of the cingulated cortex, the Medulla oblongata, Temporal lobe, Ciliary ganglion, and/or the Superior cervical ganglion.

The term "carcinoma" refers to the tissue resulting from abnormal or unregulated cell growth.

As used herein, "tumor" refers all neoplastic cell growth and proliferation, whether malignant or benign, and all pre-cancerous and cancerous cells and tissues, and particularly to an abnormal growth of cells or tissues of the malignant type. The "tumor" may be comprised of at least one cell and/or tissue.

As used herein, the term "metastasis" refers to the spread to other locations in the body, for example to another non-adjacent organ or part of an organ.

As used herein, the term "treatment" includes "therapeutic treatment" for example a curative treatment as well as "prophylactic treatment" either preventing or inhibiting the development of cancer or delaying the onset of a pre-clinically evident stage of cancer.

The terms "protein", "polypeptide", and "peptide" respectively, as used herein refer to synthetic or non-synthetic peptides, as well as purified or modified fragments of natural proteins, native forms or recombinant peptides or proteins. Moreover, the terms "protein", "polypeptide", and "peptide" respectively, as used herein refer to pharmacologic acceptable salts, pharmacologic acceptable derivatives and/or conjugates of the respective protein, polypeptide, or peptide.

The term "fragment" of a nucleic acid or amino acid sequence as used herein refers to a nucleic acid or amino acid sequence comprising a subset of the nucleic acid or amino acid sequence according to one of the claimed sequences. The same is applicable to the term "fraction" of the nucleic acid or amino acid sequence.

The term "variant" of a nucleic acid or amino acid sequence as used herein refers to a nucleic acid or amino acid sequence which is substantially similar in structure and biological activity to a nucleic acid or amino acid sequence according to one of the claimed sequences. Preferably, said term refers to a nucleic acid molecule which comprises at least one silent single nucleotide mutation (as allowed by the degeneracy of the genetic code).

The term "homologue" of a nucleic acid or amino acid sequence as used herein refers to a nucleic acid or amino acid sequence the sequence of which has one or more nucleotides or amino acids added, deleted, substituted or otherwise chemically modified in comparison to a nucleic acid or amino acid sequence according to one of the claimed sequences, provided that the homologue retains substantially the same binding properties as the latter.

The term "derivative" as used herein, refers to a nucleic acid or amino acid sequence that has similar binding characteristics to a target as a nucleic acid or amino acid sequence according to one of the claimed sequences.

The term "nucleic acid sequence" or "nucleic acid molecule" is intended to indicate any single- or double stranded nucleic acid and/or analogous molecules comprising DNA, cDNA and/or genomic DNA, RNA preferably mRNA, peptide nucleic acid (PNA), locked nucleic acid (LNA) and/or Morpholino.

The term "hybridization" as used herein is used in reference to the pairing of complementary nucleic acids. The term "stringent conditions" relates to conditions under which a nucleic acid or amino acid sequence will hybridize to its target subsequence, but to no other sequences.

The term "mutation", as used herein, is meant to refer to changes to the base pair sequence of the genetic material of an organism.

The term "tumor suppressor gene" as used herein refers to a gene that protects a cell from one step on the path to cancer. Tumor suppressor genes, or more precisely, the proteins for which they code, either have a dampening or repressive effect on the regulation of the cell cycle.

According to the present invention a pharmaceutical composition for the therapeutic and/or prophylactic treatment of cancer and/or metastasis thereof is provided comprising an amino acid sequence related to a Wech protein and/or a nucleic acid sequence of the gene encoding for the Wech protein

Surprisingly, it was found that Wech is a tumor suppressor gene that can be important in the treatment of cancer.

The term "wech" refers herein to a family of genes encoding orthologue proteins of the RING, B-box and coiled-coil/Tripartite Motif (RBCC/TRIM) protein family whose human ortholog is called Trim71, Lin41 or Lin-41. The mouse (*Mus musculus*) synonyms are called Trim-71, lin-41, LIN41, Gm1127, Ripply2 or mlin-41. The *Drosophila melanogaster* synonyms are called Dmel_CG1624, lin-41, dappled or 1(2)k08815-3. However, the inventors found that the dpld ("dpld" dappled) locus in *Drosophila melanogaster* does not correspond to CG1624 and therefore renamed CG1624 "wech".

In other words, the Wech proteins are a group of proteins belonging to the class of the RBCC/TRIM protein family. The Wech proteins are also called Lin-41 protein family.

The GenBank accession number for the human Wech gene is NM_00103911 or XM_067369. The GenBank accession number for the *Mus muscudus* Wech gene is DQ_005956. The GenBank accession number for the *Drosophida melanogaster* Wech gene is AE013599 (Flybase-ID: FBgn0015930). The databases in which the respective human and mouse genes are listed under the given access number can be accessed over the NCBI server of the US National Library of Medicine at the US National Institute of Health. The *Drosophila melanogaster* Wech gene is listed under the given access number in Flybase (http://flybase.org), a database of Drosophila Genes & Genomes.

The term "Wech protein" as used herein refers to a protein of the family of proteins of the RING, B-box and coiled-coil/Tripartite Motif (RBCC/TRIM) denoted "Wech" whose human ortholog is called Trim71, Lin41 or Lin-41. The terms "Wech polypeptide" and "Wech peptide" as used herein refer to peptides and polypeptides of the family of proteins of the RING, B-box and coiled-coil/Tripartite Motif (RBCC/TRIM) denoted "Wech" whose human ortholog is called Trim71, Lin41 or Lin-41.

The term "human Wech protein" as used herein refers to the proteins of Wech of human origin.

Preferably, the cancer is selected from the group comprising thyroid cancer, lung cancer, small cell lung cancer (SCLC), liver cancer, cancers of the atrioventricular node, cancers of the skeletal muscle, skin cancer, salivary gland cancer, ovary cancer, upper gastrointestinal cancers and/or cancers of the nervous system.

In a more preferred embodiment the cancer is selected from the group comprising cancers of the atrioventricular node, cancers of the skeletal muscle, skin cancer, salivary gland cancer, ovary cancer, and/or cancers of the nervous system.

It has been further discovered in accordance with the present invention that in particular cancer selected from the group comprising cancers of the atrioventricular node, cancers of the skeletal muscle, skin cancer, salivary gland cancer, ovary cancer, and/or cancers of the nervous system are susceptible to a treatment with an amino acid sequence related to the Wech protein and/or a nucleic acid sequence of the gene encoding for the Wech protein.

Preferred upper gastrointestinal cancers are selected from the group comprising pancreas cancer, esophagus cancer, and/or stomach cancer.

Preferred cancers of the nervous system are selected from the group comprising cancers of the cingulated cortex, the Medulla oblongata, Temporal lobe, Ciliary ganglion, and/or the Superior cervical ganglion.

In an preferred embodiment the amino acid sequence is related to the human Wech protein and/or the nucleic acid sequence of the gene encodes for the human Wech protein.

Preferably, treatment is performed in a human patient, therefore, the Wech protein and/or the nucleic acid sequence preferably is of human origin. However, even for treatment of humans it might be more preferred that the Wech protein and/or the nucleic acid sequence used is of non-human origin, for example of murine origin, or even of non-mammal origin, for example of fly, preferably Drosophila melanogaster origin.

In an preferred embodiment the amino acid sequence related to the Wech protein is a peptide, polypeptide or protein related to human Wech, wherein said peptide, polypeptide or protein
a) comprises an amino acid sequence according to SEQ ID NO: 1, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 2, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In another preferred embodiment the amino acid sequence related to the Wech protein is a peptide, polypeptide or protein related to murine Wech, wherein said peptide, polypeptide or protein
a) comprises an amino acid sequence according to SEQ ID NO: 3, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 4, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In yet another preferred embodiment the amino acid sequence related to the Wech protein is a peptide, polypeptide or protein related to fly Wech, wherein said peptide, polypeptide or protein
a) comprises an amino acid sequence according to SEQ ID NO: 5 to 7, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 8 to 10, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

It is preferred that amino acid sequences related to the Wech protein for example Wech protein, Wech peptide or Wech polypeptide may be administered since amino acid sequences generally exhibit good stability. Stable proteins or peptides can exhibit longer activity when administered.

Preferably, amino acid sequence related to the Wech protein may comprise a partial sequence of the SEQ ID NO: 1.

In a preferred embodiment the amino acid sequence related to the Wech protein is a peptide, polypeptide or protein related to human Wech, wherein said peptide, polypeptide or protein
a) comprises an amino acid sequence according to SEQ ID NO: 11, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 12, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

Advantageously, peptides, polypeptides or proteins related to human Wech, wherein said peptide, polypeptide or protein comprises an amino acid sequence according to SEQ ID NO: 11, or a fragment, variant, homologue, or derivative thereof as outlined above, will provide better specificity. Moreover, said shorter peptides, polypeptides or proteins comprising an amino acid sequence according to SEQ ID NO: 11, or a fragment, variant, homologue, or derivative thereof may provide lesser side effects on other metabolic processes influenced by the Wech protein.

In another preferred embodiment the amino acid sequence related to the Wech protein is a peptide, polypeptide or protein related to murine Wech, wherein said peptide, polypeptide or protein
a) comprises an amino acid sequence according to SEQ ID NO: 13, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 14, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In yet another preferred embodiment the amino acid sequence related to the Wech protein is a peptide, polypeptide or protein related to fly Wech, wherein said peptide, polypeptide or protein
a) comprises an amino acid sequence according to SEQ ID NO: 15, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 16, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

Advantageously, peptides, polypeptides or proteins related to murine or fly Wech, wherein said peptide, polypeptide or protein comprises an amino acid sequence according to SEQ ID NO: 13, or SEQ ID NO: 15, or a fragment, variant, homologue, or derivative thereof as outlined above, will provide better specificity. Moreover, said shorter peptides, polypeptides or proteins comprising an amino acid sequence according to SEQ ID NO: 13, or SEQ ID NO: 15, or a fragment, variant, homologue, or derivative thereof may provide lesser side effects on other metabolic processes influenced by the Wech protein.

In a more preferred embodiment the amino acid sequence of the Wech protein is a peptide of eight to twenty, preferably of nine to eighteen, more preferably of ten to sixteen, even more preferably of eleven or twelve, contiguous amino acids of a Wech protein.

In more preferred embodiment of the pharmaceutical composition the amino acid sequence related to the Wech protein is a peptide selected from the group comprising
KFGEKGTKNGQFNYPW (SEQ ID NO: 17),
CVRAHQRVRLTKDHYI (SEQ ID NO: 18),
LSLSFATEGHEDGQV (SEQ ID NO: 19) and/or
SPDSKEGSNPYKRFVHVF (SEQ ID NO: 20).

It may also be advantageously, to provide nucleic acid sequences of the gene encoding for the Wech protein.

In another preferred embodiment, the nucleic acid sequence of the gene encoding for the human Wech protein is
a) a nucleic acid sequence having the nucleotide sequence of SEQ ID NO: 2 or12, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

In another preferred embodiment, the nucleic acid sequence of the gene encoding for the murine Wech protein is
a) a nucleic acid sequence having the nucleotide sequence of SEQ ID NO: 4 or 14, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

In another preferred embodiment, the nucleic acid sequence of the gene encoding for the fly Wech protein is
a) a nucleic acid sequence having the nucleotide sequence of SEQ ID NO: 8 to 10 or 16, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

Using known sequences, proteins or nucleic acid sequences having the nucleotide sequence of a known sequence, or a fragment, variant, homologue, or derivative thereof may be synthesized using standard chemical peptide synthesis techniques. Where the desired subsequences are relatively short the molecule may be synthesized as a single contiguous polypeptide. Where larger molecules are desired, subsequences can be synthesized separately in one or more units and then fused by condensation of the amino terminus of one molecule with the carboxyl terminus of the other molecule thereby forming a peptide bond.

Solid phase synthesis in which the C-terminal amino acid of the sequence is attached to an insoluble support followed by sequential addition of the remaining amino acids in the sequence is the preferred method for the chemical synthesis of the polypeptides of this invention. Techniques for solid phase synthesis and affinity purification are described by March et al., 1974 A simplified method for cyanogen bromide activation of agarose for affinity chromatography; Anal. Biochem. Jul; 60(1):149-52.

Alternatively, proteins or nucleic acid sequences can be synthesized using recombinant DNA methodology. Generally this involves creating a DNA sequence that encodes the fusion protein, placing the DNA in an expression cassette under the control of a particular promoter, expressing the protein in a host, isolating the expressed protein and, if required, renaturing the protein.

Advantageously, the pharmaceutical composition is applied by intravenous, intraarterial, intramuscular, subcutaneous, intraperitoneal, oral, buccal, nasal, rectal, topical, transdermal, epidural, intrathecal application or locally into the tumor.

Preferred embodiments of pharmaceutical formulations comprising amino acid sequence related to the Wech protein particularly Wech peptides may comprise a solution of 0.1 M Glycerol. Other preferred embodiments of pharmaceutical formulations may comprise a salt solution, for example a solution of the peptide in Phosphate Buffered Saline (PBS) solution. Preferably, the peptide may be comprised in concentrations of 0.1 M to 1 M.

Another embodiment of the present invention provides a tumor suppressor agent comprising an amino acid sequence related to the Wech protein or a nucleic acid sequence of the gene encoding for the Wech protein according to the invention.

Surprisingly, it was found that Wech is a tumor suppressor that can be important in the treatment of cancers.

Advantageously, especially the risk of tumor metastasis can be prevented or reduced by administering Wech protein and/or the nucleic acid sequence of the gene encodes for the human Wech protein.

It is preferred that the amino acid sequence comprised by the tumor suppressor agent is related to the human Wech protein and/or the nucleic acid sequence of the gene encodes for the human Wech protein.

However, even for treatment of humans it might be more preferred that the the tumor suppressor agent comprises Wech protein and/or the nucleic acid sequence of non-human origin, for example of murine origin, or even of non-mammal origin, for example of fly, preferably Drosophila melanogaster origin.

It is to be understood that the features of the amino acid sequence related to the Wech protein and/or a nucleic acid sequence of the gene encoding for the Wech protein according to the pharmacological composition can also be features of the amino acid sequence related to the Wech protein and/or a nucleic acid sequence of the gene encoding for the Wech protein comprised by the tumor suppressor agent.

Another aspect of the present invention provides the use of an amino acid sequence related to a Wech protein and/or a nucleic acid sequence of the gene encoding for the Wech protein according to the invention as medication for therapeutic and/or prophylactic treatment of cancer and/or metastasis thereof.

Advantageously, the amino acid sequence related to the Wech protein and/or a nucleic acid sequence of the gene encoding for the Wech protein according to the invention can be used to manufacture a medication or a pharmaceutical composition for therapeutic and/or prophylactic treatment of cancer and/or metastasis thereof.

It is preferred that the cancer is selected from the group comprising thyroid cancer, lung cancer, small cell lung cancer (SCLC), liver cancer, cancers of the atrioventricular node, cancers of the skeletal muscle, skin cancer, salivary gland cancer, ovary cancer, upper gastrointestinal cancers, preferably selected from the group comprising pancreas cancer, esophagus cancer, and/or stomach cancer, and/or cancers of the nervous system, preferably selected from the group comprising cancers of the cingulated cortex, the Medulla oblongata, Temporal lobe, Ciliary ganglion, and/or the Superior cervical ganglion.

Preferably, the Wech protein and/or the nucleic acid sequence is used as a pharmaceutical agent in humans. Therefore, the Wech protein and/or the nucleic acid sequence used preferably is of human origin. However, even for treatment of humans is might be more preferred that the Wech protein and/or the nucleic acid sequence used is of non-human origin, for example of murine origin, or even of non-mammal origin, for example of fly, preferably Drosophila melanogaster origin.

It is preferred for the use of an amino acid sequence related to the Wech protein and/or a nucleic acid sequence of the gene encoding for the Wech protein as medication for therapeutic and/or prophylactic treatment of cancer and/or metastasis thereof that the amino acid sequence related to the Wech protein is a peptide, polypeptide or protein related to human Wech, wherein said peptide, polypeptide or protein
a) comprises an amino acid sequence according to SEQ ID NO: 1, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 2, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In another preferred embodiment of the use as medication the amino acid sequence related to the Wech protein is a peptide, polypeptide or protein related to murine Wech, wherein said peptide, polypeptide or protein
a) comprises an amino acid sequence according to SEQ ID NO: 3, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 4, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In yet another preferred embodiment of the use as medication the amino acid sequence related to the Wech protein is a peptide, polypeptide or protein related to fly Wech, wherein said peptide, polypeptide or protein
a) comprises an amino acid sequence according to SEQ ID NO: 5 to 7, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 8 to 10, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

Preferably, usable amino acid sequence related to the Wech protein may comprise a partial sequence of the SEQ ID NO: 1, SEQ ID NO: 3 or SEQ ID NO: 5 to 7.

In a preferred embodiment of the use as medication the amino acid sequence related to the Wech protein is a peptide, polypeptide or protein related to human Wech, wherein said peptide, polypeptide or protein
a) comprises an amino acid sequence according to SEQ ID NO: 11, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 12, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In another preferred embodiment of the use as medication the amino acid sequence related to the Wech protein is a peptide, polypeptide or protein related to murine Wech, wherein said peptide, polypeptide or protein
a) comprises an amino acid sequence according to SEQ ID NO: 13, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 14, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

In yet another preferred embodiment of the use as medication the amino acid sequence related to the Wech protein is a peptide, polypeptide or protein related to fly Wech, wherein said peptide, polypeptide or protein
a) comprises an amino acid sequence according to SEQ ID NO: 15, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 16, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

Advantageously, peptides, polypeptides or proteins related to human, murine or fly Wech, wherein said peptide, polypeptide or protein comprises an amino acid sequence according to SEQ ID NO: 11, SEQ ID NO: 13, or SEQ ID NO: 15, or a fragment, variant, homologue, or derivative thereof as outlined above, will provide better specificity. Moreover, said shorter peptides, polypeptides or proteins comprising an amino acid sequence according to SEQ ID NO: 11, SEQ ID NO: 13, or SEQ ID NO: 15, or a fragment, variant, homologue, or derivative thereof may provide lesser side effects on other metabolic processes influenced by the Wech protein.

In more preferred embodiment of the use as medication the amino acid sequence related to the Wech protein is a peptide selected from the group comprising
KFGEKGTKNGQFNYPW (SEQ ID NO: 17),
CVRAHQRVRLTKDHYI (SEQ ID NO: 18),
LSLSFATEGHEDGQV (SEQ ID NO: 19) and/or
SPDSKEGSNPYKRFVHVF (SEQ ID NO: 20).

Advantageously, peptides selected from the group comprising SEQ ID NO: 17, SEQ ID NO: 18, SEQ ID NO: 19 and/or SEQ ID NO: 20, will provide even better specificity and/or lesser side effects on other metabolic processes influenced by the Wech protein.

It is to be understood that the features of the amino acid sequence related to the Wech protein and/or a nucleic acid sequence of the gene encoding for the Wech protein according to the pharmacological composition can also be features of the amino acid sequence related to the Wech protein and/or a nucleic acid sequence of the gene encoding for the Wech protein for the use as medication according the present invention.

It may also be advantageously, to provide nucleic acid sequences of the gene encoding for the Wech protein for use of the amino acid sequence related to the Wech protein and/or a nucleic acid sequence of the gene encoding for the Wech protein as a medication.

In a preferred embodiment, of the use as medication the nucleic acid sequence of the gene encoding for the human Wech protein is
a) a nucleic acid sequence having the nucleotide sequence of SEQ ID NO: 2 or 12, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

In another preferred embodiment of the use as a medication, the nucleic acid sequence of the gene encoding for the murine Wech protein is
a) a nucleic acid sequence having the nucleotide sequence of SEQ ID NO: 4 or 14, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

In another preferred embodiment of the use as a medication, the nucleic acid sequence of the gene encoding for the fly Wech protein is
a) a nucleic acid sequence having the nucleotide sequence of SEQ ID NO: 8 to 10 or 16, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

Another embodiment of the present invention provides peptides, pharmacologic acceptable salts, derivatives and/or conjugates thereof selected from the group comprising
CVRAHQRVRLTKDHYI (SEQ ID NO: 18), and/or
SPDSKEGSNPYKRFVHVF (SEQ ID NO: 20).

Advantageously, peptides selected from the group comprising SEQ ID NO: 18 and/or SEQ ID NO: 20, showed better specificity and/or lesser side effects on other metabolic processes influenced by the Wech protein.

Further features of the present invention will become apparent from the examples, which, in an exemplary fashion, show preferred embodiments of the present invention. However, these examples should by no means be understood as to limit the scope of the invention.

### Example 1

### A pharmaceutical composition comprising a Wech peptide

An aqueous solution of 0.1 M Glycerol of pH 2.4 comprising 1 M of a peptide having the following sequence as set forth in SEQ ID NO: 11

LCRPWGVSVDKEGYIIVADRSNNRIQVFKPCGAFHHKFGTLGSRPGQFDRPAGVACD
ASRRIVVADKDNHRIQIFTFEGQFLLKFGEKGTKNGQFNYPWDVAVNSEGKILVSDT
RNHRIQLFGPDGVFLNKYGFEGALWKHFDSPRGVAFNHEGHLVVTDFNNHRLLVIHP
DCQSARFLGSEGTGNGQFLRPQGVAVDQEGRIIVADSRNHRVQMFESNGSFLCKFGA
QGSGFGQMDRPSGIAITPDGMIVVVDFGNNRILVF (SEQ ID NO: 11)

### Example 2

### A pharmaceutical composition comprising a Wech peptide

An aqueous solution of 0.1 M Glycerol of pH 2.4 comprising 1 M of a peptide having the following sequence as set forth in SEQ ID NO: 13

LCRPWGVSVDKEGFIIVADRSNNRIQVFKPCGSFHHKFGTLGSRPGQFDRPAGVACD
ASRRIIVADKDNHRIQIFTFEGQFLLKFGEKGTKNGQFNYPWDVAVNSEGKILVSDTR
NHRIQLFGPDGVFLNKYGFEGSLWKHFDSPRGVAFNNEGHLVVTDFNNHRLLVIHPD
CQSARFLGSEGSGNGQFLRPQGVAVDQEGRIIVADSRNHRVQMFEANGSFLCKFGAQ
G SGFGQMDRPSGIAVTPDGLIVVVDFGNNRILIF (SEQ ID NO: 13).

### Example 3

### A pharmaceutical composition comprising a Wech peptide

An aqueous solution of Phosphate Buffered Saline (PBS) buffer comprising 130 mM NaCl, 7 mM Na₂HPO₄, 3 mM NaH₂PO4 of pH 7.2 comprising 0,1 M of a peptide having the following sequence as set forth in SEQ ID NO: 17: KFGEKGTKNGQFNYPW (SEQ ID NO: 17).

### Example 4

### A pharmaceutical composition comprising a Wech peptide

An aqueous solution of Phosphate Buffered Saline (PBS) buffer comprising 130 mM NaCl, 7 mM Na₂HPO₄, 3 mM NaH₂PO4 of pH 7.2 comprising 1 M of a peptide having the following sequence as set forth in SEQ ID NO: 18: CVRAHQRVRLTKDHYI (SEQ ID NO: 18).

### Example 5

### A pharmaceutical composition comprising a Wech peptide

An aqueous solution of 0.1 M Glycerol of pH 2.4 comprising 1 M of a peptide having the following sequence as set forth in SEQ ID NO: 15

VSRPWGLCVDKMGHVLVSDRRNNRVQVFNPDGSLKFKFGRKGVGNGEFDLPAGIC
VDVDNRIIVVDKDNHRVQIFTASGVFLLKFGSYGKEYGQFQYPWDVAVNSRRQIVVT
DSRNHRIQQFDSEGRFIRQIVFDNHGQTKGIASPRGVCYTPTGNIIVSDFDNHCLYLIDP
DINDILSVKGHEGSGFHEFNRPSGLCCDDEGRIIVADSKNQRILVFNQNLDFMWDIEV
RPSINPLMPPTLDEKDRTCDVAIMPDGRIVFLIELSPDSKEGSNPYKRFVHVF (SEQ ID
NO: 15).

### Example 6

### A pharmaceutical composition comprising a Wech peptide

An aqueous solution of Phosphate Buffered Saline (PBS) buffer comprising 130 mM NaCl, 7 mM Na₂HPO₄, 3 mM NaH₂PO4 of pH 7.2 comprising 0,1 M of a peptide having the following sequence as set forth in SEQ ID NO: 19: LSLSFATEGHEDGQV (SEQ ID NO: 19).

### Example 7

### A pharmaceutical composition comprising a Wech peptide

An aqueous solution of Phosphate Buffered Saline (PBS) buffer comprising 130 mM NaCl, 7 mM Na₂HPO₄, 3 mM NaH₂PO4 of pH 7.2 comprising 0,1 M of a peptide having the following sequence as set forth in SEQ ID NO: 20: SPDSKEGSNPYKRFVHVF (SEQ ID NO: 20).

It is to be understood that this invention is not limited to the particular features of the composition described as such means and methods may vary. It is also to be understood that the terminology used herein is for purposes of describing particular embodiments only, and is not intended to be limiting. It must be noted that, as used in the specification and the appended claims, the singular forms "a" "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

The particular combinations of elements and features in the above detailed embodiments are exemplary only. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

## Claims

1. A pharmaceutical composition for the therapeutic and/or prophylactic treatment of cancer and/or metastasis thereof comprising an amino acid sequence related to a Wech protein and/or a nucleic acid sequence of the gene encoding for the Wech protein.

2. The pharmaceutical composition according to claim 1, **characterized in that** the cancer is selected from the group comprising thyroid cancer, lung cancer, small cell lung cancer (SCLC), liver cancer, cancers of the atrioventricular node, cancers of the skeletal muscle, skin cancer, salivary gland cancer, ovary cancer, upper gastrointestinal cancers and/or cancers of the nervous system.

3. The pharmaceutical composition according to claims 1 or 2, **characterized in that** the upper gastrointestinal cancers are selected from the group comprising pancreas cancer, esophagus cancer, and/or stomach cancer.

4. The pharmaceutical composition according to any one of the aforementioned claims, **characterized in that** the cancers of the nervous system are selected from the group comprising cancers of the cingulated cortex, the Medulla oblongata, Temporal lobe, Ciliary ganglion, and/or the Superior cervical ganglion.

5. The pharmaceutical composition according to any one of the aforementioned claims, **characterized in that** the amino acid sequence related to the Wech protein is a peptide, polypeptide or protein related to human Wech, wherein said peptide, polypeptide or protein
a) comprises an amino acid sequence according to SEQ ID NO: 1, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 2, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

6. The pharmaceutical composition according to any one of the aforementioned claims, **characterized in that** the amino acid sequence related to the Wech protein is a peptide, polypeptide or protein related to human Wech, wherein said peptide, polypeptide or protein
a) comprises an amino acid sequence according to SEQ ID NO: 11, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 12, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

7. The pharmaceutical composition according to any one of the aforementioned claims, **characterized in that** the amino acid sequence of the Wech protein is a peptide of eight to twenty, preferably of nine to eighteen, more preferably of ten to sixteen, even more preferably of eleven or twelve, contiguous amino acids of a Wech protein.

8. The pharmaceutical composition according to any one of the aforementioned claims, **characterized in that** the nucleic acid sequence of the gene encoding for the human Wech protein is
a) a nucleic acid sequence having the nucleotide sequence of SEQ ID NO: 2 or 12, or a fragment, variant, homologue, or derivative thereof,
b) a nucleic acid sequence having a sequence identity of at least 70, preferably 85 %, more preferably 95 % with any of the nucleic acid sequences of a),
c) a nucleic acid molecule which comprises, in comparison to the nucleic acid molecule according to a) to b) at least one silent single nucleotide mutation (as allowed by the degeneracy of the genetic code), and/or
d) a nucleic acid molecule which, in comparison to the nucleic acid molecule according to a) to c), is code optimized for a given expression host.

9. Tumor suppressor agent comprising an amino acid sequence related to a Wech protein and/or a nucleic acid sequence of the gene encoding for the Wech protein according to any one of the aforementioned claims.

10. Use of an amino acid sequence related to a Wech protein and/or a nucleic acid sequence of the gene encoding for the Wech protein according to any one of the aforementioned claims as medication for therapeutic and/or prophylactic treatment of cancer and/or metastasis thereof.

11. The use according to claim 10, **characterized in that** the cancer is selected from the group comprising thyroid cancer, lung cancer, small cell lung cancer (SCLC), liver cancer, cancers of the atrioventricular node, cancers of the skeletal muscle, skin cancer, salivary gland cancer, ovary cancer, upper gastrointestinal cancers, preferably selected from the group comprising pancreas cancer, esophagus cancer, and/or stomach cancer, and/or cancers of the nervous system, preferably selected from the group comprising cancers of the cingulated cortex, the Medulla oblongata, Temporal lobe, Ciliary ganglion, and/or the Superior cervical ganglion.

12. The use according to claim 10 or 11, **characterized in that** the amino acid sequence related to the Wech protein is a peptide, polypeptide or protein related to human Wech, wherein said peptide, polypeptide or protein
a) comprises an amino acid sequence according to SEQ ID NO: 1, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 2, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

13. The use according to any one of the aforementioned claims, **characterized in that** the amino acid sequence related to the Wech protein is a peptide, polypeptide or protein related to human Wech, wherein said peptide, polypeptide or protein
a) comprises an amino acid sequence according to SEQ ID NO: 11, or a fragment, variant, homologue, or derivative thereof,
b) comprises an amino acid sequence having a sequence identity of at least 70, preferably 85 %, more preferably 95 % with any of the amino acid sequence of a),
c) is encoded by the nucleic acid of SEQ ID NO: 12, or a fragment, variant, homologue or derivative thereof,
d) is encoded by a nucleic acid molecule that is capable of hybridizing to any of the nucleic acid molecules of c) under stringent conditions,
e) is encoded by a nucleic acid molecule having a sequence identity of at least 70, preferably 85 %, more preferably 95 % with any of the nucleic acid molecules of c) or d),
f) is encoded by a nucleic acid molecule that is capable of hybridizing to the complement of any of the nucleic acid molecules of c) to e) under stringent conditions,
g) is encoded by a nucleic acid molecule which comprises, in comparison to the nucleic acid molecule according to c) to f) at least one silent single nucleotide mutation (as allowed by the degeneracy of the genetic code), and/or
h) is encoded by a nucleic acid molecule which, in comparison to the nucleic acid molecule according to c) to g), is code optimized for a given expression host.

14. The use according to any one of the aforementioned claims, **characterized in that** the amino acid sequence related to the Wech protein is a peptide selected from the group comprising
KFGEKGTKNGQFNYPW (SEQ ID NO: 17),
CVRAHQRVRLTKDHYI (SEQ ID NO: 18),
LSLSFATEGHEDGQV (SEQ ID NO: 19) and/or
SPDSKEGSNPYKRFVHVF (SEQ ID NO: 20).

15. Peptides, pharmacologic acceptable salts, derivatives and/or conjugates thereof selected from the group comprising
CVRAHQRVRLTKDHYI (SEQ ID NO: 18), and/or
SPDSKEGSNPYKRFVHVF (SEQ ID NO: 20).


SEQUENCE LISTING

<110>  Rheinische Friedrich-Wilhelms Universität

<120>  Anticancer agent

<130>  UD 40074/SAM

<160>  20

<170>  PatentIn version 3.3

<210>  1
<211>  868
<212>  PRT
<213>  human

<400>  1

Met Ala Ser Phe Pro Glu Thr Asp Phe Gln Ile Cys Leu Leu Cys Lys
1               5                   10                  15

Glu Met Cys Gly Ser Pro Ala Pro Leu Ser Ser Asn Ser Ser Ala Ser
            20                  25                  30

Ser Ser Ser Ser Gln Thr Ser Thr Ser Ser Gly Gly Gly Gly Gly Gly
        35                  40                  45

Pro Gly Ala Ala Ala Arg Arg Leu His Val Leu Pro Cys Leu His Ala
    50                  55                  60

Phe Cys Arg Pro Cys Leu Glu Ala His Arg Leu Pro Ala Ala Gly Gly
65                  70                  75                  80

Gly Ala Ala Gly Glu Pro Leu Lys Leu Arg Cys Pro Val Cys Asp Gln
                85                  90                  95

Lys Val Val Leu Ala Glu Ala Ala Gly Met Asp Ala Leu Pro Ser Ser
            100                 105                 110

Ala Phe Leu Leu Ser Asn Leu Leu Asp Ala Val Val Ala Thr Ala Asp
        115                 120                 125

Glu Pro Pro Pro Lys Asn Gly Arg Ala Gly Ala Pro Ala Gly Ala Gly
    130                 135                 140

Gly His Ser Asn His Arg His His Ala His His Ala His Pro Arg Ala
145                 150                 155                 160

Ser Ala Ser Ala Pro Pro Leu Pro Gln Ala Pro Gln Pro Pro Ala Pro
                165                 170                 175

Ser Arg Ser Ala Pro Gly Gly Pro Ala Ala Ser Pro Ser Ala Leu Leu
            180                 185                 190

Leu Arg Arg Pro His Gly Cys Ser Ser Cys Asp Glu Gly Asn Ala Ala

        195                 200                 205

Ser Ser Arg Cys Leu Asp Cys Gln Glu His Leu Cys Asp Asn Cys Val
    210                 215                 220

Arg Ala His Gln Arg Val Arg Leu Thr Lys Asp His Tyr Ile Glu Arg
225                 230                 235                 240

Gly Pro Pro Gly Pro Gly Ala Ala Ala Ala Ala Gln Gln Leu Gly Leu
                245                 250                 255

Gly Pro Pro Phe Pro Gly Pro Pro Phe Ser Ile Leu Ser Val Phe Pro
            260                 265                 270

Glu Arg Leu Gly Phe Cys Gln His His Asp Asp Glu Val Leu His Leu
        275                 280                 285

Tyr Cys Asp Thr Cys Ser Val Pro Ile Cys Arg Glu Cys Thr Met Gly
    290                 295                 300

Arg His Gly Gly His Ser Phe Ile Tyr Leu Gln Glu Ala Leu Gln Asp
305                 310                 315                 320

Ser Arg Ala Leu Thr Ile Gln Leu Leu Ala Asp Ala Gln Gln Gly Arg
                325                 330                 335

Gln Ala Ile Gln Leu Ser Ile Glu Gln Ala Gln Thr Val Ala Glu Gln
            340                 345                 350

Val Glu Met Lys Ala Lys Val Val Gln Ser Glu Val Lys Ala Val Thr
        355                 360                 365

Ala Arg His Lys Lys Ala Leu Glu Glu Arg Glu Cys Glu Leu Leu Trp
    370                 375                 380

Lys Val Glu Lys Ile Arg Gln Val Lys Ala Lys Ser Leu Tyr Leu Gln
385                 390                 395                 400

Val Glu Lys Leu Arg Gln Asn Leu Asn Lys Leu Glu Ser Thr Ile Ser
                405                 410                 415

Ala Val Gln Gln Val Leu Glu Glu Gly Arg Ala Leu Asp Ile Leu Leu
            420                 425                 430

Ala Arg Asp Arg Met Leu Ala Gln Val Gln Glu Leu Lys Thr Val Arg
        435                 440                 445

Ser Leu Leu Gln Pro Gln Glu Asp Asp Arg Val Met Phe Thr Pro Pro
    450                 455                 460

Asp Gln Ala Leu Tyr Leu Ala Ile Lys Ser Phe Gly Phe Val Ser Ser

465                 470                 475                 480

Gly Ala Phe Ala Pro Leu Thr Lys Ala Thr Gly Asp Gly Leu Lys Arg
                485                 490                 495

Ala Leu Gln Gly Lys Val Ala Ser Phe Thr Val Ile Gly Tyr Asp His
            500                 505                 510

Asp Gly Glu Pro Arg Leu Ser Gly Gly Asp Leu Met Ser Ala Val Val
        515                 520                 525

Leu Gly Pro Asp Gly Asn Leu Phe Gly Ala Glu Val Ser Asp Gln Gln
    530                 535                 540

Asn Gly Thr Tyr Val Val Ser Tyr Arg Pro Gln Leu Glu Gly Glu His
545                 550                 555                 560

Leu Val Ser Val Thr Leu Cys Asn Gln His Ile Glu Asn Ser Pro Phe
                565                 570                 575

Lys Val Val Val Lys Ser Gly Arg Ser Tyr Val Gly Ile Gly Leu Pro
            580                 585                 590

Gly Leu Ser Phe Gly Ser Glu Gly Asp Ser Asp Gly Lys Leu Cys Arg
        595                 600                 605

Pro Trp Gly Val Ser Val Asp Lys Glu Gly Tyr Ile Ile Val Ala Asp
    610                 615                 620

Arg Ser Asn Asn Arg Ile Gln Val Phe Lys Pro Cys Gly Ala Phe His
625                 630                 635                 640

His Lys Phe Gly Thr Leu Gly Ser Arg Pro Gly Gln Phe Asp Arg Pro
                645                 650                 655

Ala Gly Val Ala Cys Asp Ala Ser Arg Arg Ile Val Val Ala Asp Lys
            660                 665                 670

Asp Asn His Arg Ile Gln Ile Phe Thr Phe Glu Gly Gln Phe Leu Leu
        675                 680                 685

Lys Phe Gly Glu Lys Gly Thr Lys Asn Gly Gln Phe Asn Tyr Pro Trp
    690                 695                 700

Asp Val Ala Val Asn Ser Glu Gly Lys Ile Leu Val Ser Asp Thr Arg
705                 710                 715                 720

Asn His Arg Ile Gln Leu Phe Gly Pro Asp Gly Val Phe Leu Asn Lys
                725                 730                 735

Tyr Gly Phe Glu Gly Ala Leu Trp Lys His Phe Asp Ser Pro Arg Gly

740                 745                 750

Val Ala Phe Asn His Glu Gly His Leu Val Val Thr Asp Phe Asn Asn
        755                 760                 765

His Arg Leu Leu Val Ile His Pro Asp Cys Gln Ser Ala Arg Phe Leu
    770                 775                 780

Gly Ser Glu Gly Thr Gly Asn Gly Gln Phe Leu Arg Pro Gln Gly Val
785                 790                 795                 800

Ala Val Asp Gln Glu Gly Arg Ile Ile Val Ala Asp Ser Arg Asn His
                805                 810                 815

Arg Val Gln Met Phe Glu Ser Asn Gly Ser Phe Leu Cys Lys Phe Gly
            820                 825                 830

Ala Gln Gly Ser Gly Phe Gly Gln Met Asp Arg Pro Ser Gly Ile Ala
        835                 840                 845

Ile Thr Pro Asp Gly Met Ile Val Val Val Asp Phe Gly Asn Asn Arg
    850                 855                 860

Ile Leu Val Phe
865

<210>  2
<211>  3138
<212>  DNA
<213>  human

<400>  2
gctctctcct cctcctcctc ctcttcctct ctggtctcct ccctcctccg ggctgggttg      60

caaatggctt cgttccccga gaccgatttc cagatctgct tgctgtgcaa ggagatgtgc     120

ggctcgccgg cgccgctctc ctccaactcg tccgcgtcgt cgtcctcctc gcagacgtcc     180

acgtcgtcgg ggggcggcgg cgggggccct ggggcggcgg cgcgccgcct acacgtcctg     240

ccctgcctgc acgccttctg ccgcccctgc ctcgaggcgc accggctgcc ggcggcgggc     300

ggcggcgcgg cgggagagcc gctcaagctg cgctgccccg tgtgcgacca gaaagtagtg     360

ctagccgagg cggcgggtat ggacgcgctg ccttcgtccg ccttcctgct tagcaacctg     420

ctcgacgcgg tggtggccac tgccgacgag ccgccgccca agaacgggcg cgccggcgct     480

ccggcgggag cgggcggcca cagcaaccac cggcaccacg ctcaccacgc gcacccgcgc     540

gcgtccgcct ccgcgccgcc actcccgcag gcgccgcagc cgcccgcgcc ttcccgctcg     600

gcacccggcg gccctgccgc ttccccgtcg gcgctgctgc tccgccgtcc tcacggctgc     660

agctcgtgcg atgagggcaa cgcagcttct tcgcgctgcc tcgactgcca ggagcacctg     720

tgcgacaact gcgtccgagc gcaccagcgc gtgcgcctca ccaaggacca ctacatcgag     780

cgcggcccgc cgggtcccgg tgccgcagca gcggcgcagc agctcgggct cggcccgccc     840

tttcccggcc cgcccttctc catcctctca gtgtttcccg agcgcctcgg cttctgccag 900
caccacgacg acgaggtgct gcacctgtac tgtgacactt gctctgtacc catctgtcgt 960
gagtgcacaa tgggccggca tgggggccac agcttcatct acctccagga ggcactgcag 1020
gactcacggg cactcaccat ccagctgctg gcagatgccc agcagggacg acaggcaatc 1080
cagctgagca tcgagcaggc ccagacggtg gcggaacagg tggagatgaa ggcgaaggtt 1140
gtgcagtcgg aggtcaaagc cgtgacggcg aggcataaga aagccctgga ggaacgcgag 1200
tgtgagctgc tgtggaaggt agaaaagatc cgccaggtga aagccaagtc tctgtacctg 1260
caggtggaga agctgcggca aaacctcaac aagcttgaga gcaccatcag tgccgtgcag 1320
caggtcctgg aggagggtag agcgctagac atcctactgg cccgagaccg gatgctggcc 1380
caggtgcagg agctgaagac cgtgcggagc ctcctgcagc cccaggaaga cgaccgagtc 1440
atgttcacac cccccgatca ggcactgtac cttgccatca agtcttttgg ctttgttagc 1500
agcggggcct ttgccccact caccaaggcc acaggcgatg gcctcaagcg tgccctccag 1560
ggtaaggtgg cctccttcac agtcattggt tatgaccacg atggtgagcc ccgcctctca 1620
ggaggcgacc tgatgtcggc tgtggtcctg ggccctgatg gcaacctgtt tggtgcagag 1680
gtgagtgatc agcagaatgg gacatacgtg gtgagttacc gaccccagct ggagggtgag 1740
cacctggtat ctgtgacact gtgcaaccag cacattgaga acagcccttt caaggtggtg 1800
gtcaagtcag gccgcagcta cgtgggcatt gggctcccgg gcctgagctt cggcagtgag 1860
ggtgacagcg atggcaagct ctgccgccct tggggtgtga gtgtagacaa ggagggctac 1920
atcattgtcg ccgaccgcag caacaaccgc atccaggtgt tcaagccctg cggcgccttc 1980
caccacaaat tcggcaccct gggctcccgg cctgggcagt tcgaccgacc agccggcgtg 2040
gcctgtgacg cctcacgcag gatcgtggtg gctgacaagg acaatcatcg catccagatc 2100
ttcacgttcg agggccagtt cctcctcaag tttggtgaga aaggaaccaa gaatgggcag 2160
ttcaactacc cttgggatgt ggcggtgaat tctgagggca agatcctggt ctcagacacg 2220
aggaaccacc ggatccagct gtttgggcct gatggtgtct tcctaaacaa gtatggcttc 2280
gagggggctc tctggaagca ctttgactcc ccacggggtg tggccttcaa ccatgagggc 2340
cacttggtgg tcactgactt caacaaccac cggctcctgg ttattcaccc cgactgccag 2400
tcggcacgct ttctgggctc ggagggcaca ggcaatgggc agttcctgcg cccacaaggg 2460
gtagctgtgg accaggaagg gcgcatcatt gtggcggatt ccaggaacca tcgggtacag 2520
atgtttgaat ccaacggcag cttcctgtgc aagtttggtg ctcaaggcag cggctttggg 2580
cagatggacc gcccttccgg catcgccatc acccccgacg gaatgatcgt tgtggtggac 2640
tttggcaaca atcgaatcct cgtcttctaa ttgcatttcc taggtttctg tgtttggggt 2700
gtgtgtgcgt gtctctctct ctctctctct ctttctcttt ctctctcttt ttgaatttca 2760
aagaagaaac agtctcaggg aaatttcttt tttctttttt ttttttaaag agaacaagaa 2820
aagtacaaca ttgcttaagt cctacctcat ctttattttt ttacagatga atgtacttat 2880

cttttctgca gggattgagc ctgtgaagtg ataatttcta tctacctcat aaatctttac 2940

atttccttct gcaacaggcc ctcttcccct cctcagtgga gtttgcattt ccctcttccc 3000

ctgcgtgggg catgatatgc acaagcctgg catctgtatg gctgggaggg cactggatgt 3060

gtgtggtggg gtgtattctg tagattgagc caaggaaaca caaaaaaaaa ctactaagta 3120

aaaaaacaaa aaactata 3138

<210> 3
<211> 855
<212> PRT
<213> Mus musculus

<400> 3

Met Ala Ser Phe Pro Glu Thr Asp Phe Gln Ile Cys Leu Leu Cys Lys
1               5                   10                  15

Glu Met Cys Gly Ser Pro Ala Pro Leu Ser Ser Asn Ser Ser Ala Ser
            20                  25                  30

Ser Ser Ser Ser Gln Thr Ser Thr Ser Ser Ala Gly Gly Gly Gly Pro
        35                  40                  45

Gly Ala Ala Ala Arg Arg Leu His Val Leu Pro Cys Leu His Ala Phe
    50                  55                  60

Cys Arg Pro Cys Leu Glu Ala His Arg Leu Pro Ala Pro Gly Gly Ala
65                  70                  75                  80

Gly Pro Ala Glu Ala Leu Lys Leu Arg Cys Pro Val Cys Asp Gln Lys
                85                  90                  95

Val Val Leu Ala Glu Ala Ala Gly Met Asp Ala Leu Pro Ser Ser Ala
            100                 105                 110

Phe Leu Leu Ser Asn Leu Leu Asp Ala Val Val Ala Thr Ala Glu Glu
        115                 120                 125

Pro Pro Pro Lys Asn Gly Arg Ala Gly Gly Gly Pro Gly Gly Ala Gly
    130                 135                 140

Gly His Ser Asn His Arg His His Ala His His Pro Ala Gln Arg Ala
145                 150                 155                 160

Ala Ala Pro Ala Pro Gln Pro Pro Pro Gly Pro Ala Ala Ser Pro Gly
                165                 170                 175

Ser Leu Leu Met Arg Arg Pro His Gly Cys Ser Ser Cys Asp Glu Gly
            180                 185                 190

Asn Ala Ala Ser Ser Arg Cys Leu Asp Cys Gln Glu His Leu Cys Asp

        195                 200                 205

Asn Cys Val Arg Ala His Gln Arg Val Arg Leu Thr Lys Asp His Tyr
    210                 215                 220

Ile Glu Arg Gly Pro Pro Gly Pro Ala Ala Ala Ser Ala Ala Gln Gln
225                 230                 235                 240

Leu Gly Leu Gly Pro Pro Phe Ala Gly Ala Pro Phe Ser Ile Leu Ser
                245                 250                 255

Val Phe Pro Glu Arg Leu Gly Phe Cys Gln His His Asp Asp Glu Val
            260                 265                 270

Leu His Leu Tyr Cys Asp Thr Cys Ser Val Pro Ile Cys Arg Glu Cys
        275                 280                 285

Thr Leu Gly Arg His Gly Gly His Ser Phe Ala Tyr Leu Gln Asp Ala
    290                 295                 300

Leu Gln Asp Ser Arg Ala Leu Thr Ile Gln Leu Leu Ala Asp Ala Gln
305                 310                 315                 320

Gln Gly Arg Gln Ala Leu Gln Leu Ser Ile Glu Gln Ala Gln Thr Val
                325                 330                 335

Ala Glu Gln Val Glu Met Lys Ala Lys Val Val Gln Ser Glu Val Lys
            340                 345                 350

Ala Val Thr Ala Arg His Lys Lys Ala Leu Glu Asp Arg Glu Cys Glu
        355                 360                 365

Leu Leu Trp Lys Val Glu Lys Ile Arg Gln Val Lys Ala Lys Ser Leu
    370                 375                 380

Phe Leu Gln Val Glu Lys Leu Arg Gln Ser Leu Ser Lys Leu Glu Ser
385                 390                 395                 400

Thr Ile Ser Ala Val Gln Gln Val Leu Glu Glu Gly Arg Ala Leu Asp
                405                 410                 415

Ile Leu Leu Ala Arg Asp Arg Met Leu Ala Gln Val Gln Glu Leu Lys
            420                 425                 430

Thr Ile Arg Gly Leu Leu Gln Pro Gln Glu Asp Asp Arg Ile Met Phe
        435                 440                 445

Thr Pro Pro Asp Gln Ala Leu Tyr Leu Ala Leu Lys Ser Ile Gly Phe
    450                 455                 460

Val Ser Ser Gly Ala Phe Ala Pro Leu Thr Lys Ala Thr Gly Asp Gly

465                 470                 475                 480

Ile Lys Arg Ala Leu Gln Gly Lys Val Ala Ser Phe Thr Val Met Gly
                485                 490                 495

Tyr Asp His Asp Gly Glu Pro Arg His Ser Gly Gly Asp Leu Met Ser
            500                 505                 510

Val Val Val Leu Gly Pro Asp Gly Asn Leu Phe Gly Ala Glu Val Ser
        515                 520                 525

Asp Gln Gln Asn Gly Thr Tyr Ile Val Ser Tyr Arg Pro Gln Leu Glu
    530                 535                 540

Gly Glu His Leu Val Ser Val Thr Leu Tyr Asn Gln His Ile Glu Asn
545                 550                 555                 560

Ser Pro Phe Lys Val Val Val Lys Ser Gly Arg Ser Tyr Val Gly Ile
                565                 570                 575

Gly Leu Pro Gly Leu Ser Phe Gly Ser Glu Gly Asp Gly Glu Gly Lys
            580                 585                 590

Leu Cys Arg Pro Trp Gly Val Ser Val Asp Lys Glu Gly Phe Ile Ile
        595                 600                 605

Val Ala Asp Arg Ser Asn Asn Arg Ile Gln Val Phe Lys Pro Cys Gly
    610                 615                 620

Ser Phe His His Lys Phe Gly Thr Leu Gly Ser Arg Pro Gly Gln Phe
625                 630                 635                 640

Asp Arg Pro Ala Gly Val Ala Cys Asp Ala Ser Arg Arg Ile Ile Val
                645                 650                 655

Ala Asp Lys Asp Asn His Arg Ile Gln Ile Phe Thr Phe Glu Gly Gln
            660                 665                 670

Phe Leu Leu Lys Phe Gly Glu Lys Gly Thr Lys Asn Gly Gln Phe Asn
        675                 680                 685

Tyr Pro Trp Asp Val Ala Val Asn Ser Glu Gly Lys Ile Leu Val Ser
    690                 695                 700

Asp Thr Arg Asn His Arg Ile Gln Leu Phe Gly Pro Asp Gly Val Phe
705                 710                 715                 720

Leu Asn Lys Tyr Gly Phe Glu Gly Ser Leu Trp Lys His Phe Asp Ser
                725                 730                 735

Pro Arg Gly Val Ala Phe Asn Asn Glu Gly His Leu Val Val Thr Asp

740                 745                 750

Phe Asn Asn His Arg Leu Leu Val Ile His Pro Asp Cys Gln Ser Ala
        755                 760                 765

Arg Phe Leu Gly Ser Glu Gly Ser Gly Asn Gly Gln Phe Leu Arg Pro
    770                 775                 780

Gln Gly Val Ala Val Asp Gln Glu Gly Arg Ile Ile Val Ala Asp Ser
785                 790                 795                 800

Arg Asn His Arg Val Gln Met Phe Glu Ala Asn Gly Ser Phe Leu Cys
                805                 810                 815

Lys Phe Gly Ala Gln Gly Ser Gly Phe Gly Gln Met Asp Arg Pro Ser
            820                 825                 830

Gly Ile Ala Val Thr Pro Asp Gly Leu Ile Val Val Val Asp Phe Gly
        835                 840                 845

Asn Asn Arg Ile Leu Ile Phe
    850                 855

<210>  4
<211>  5186
<212>  DNA
<213>  Mus musculus

<400>  4
gtttgctcct ttggagatta aaaggattcc gaattccgat cagtcccaat aacccgcgtt      60

ctgagaggcg gcaaagcgga gaaagaaggg ggacacgcgc acgcacacag cccctcgttc     120

gggggagtcg tgcaaaataa agcccaaagc aacccaggac accagggcat cgggagagcc     180

tgagcccggt aggcgcagcg cagccggggc cacccgcatc cccgctcggc gaatcgttcg     240

taggaggatg acgcaggcgg gagccgcggg cggcgttggc cacgcggctc ccgaagtggc     300

cgcgcgggtc cctcgcgtac tcctctgatt cgagcaagcc cgggagggag actcggttcc     360

cgaggctccg ggattgaagg ggggacccaa gcgagggtga aatggcccga tgacaaagga     420

aatgtgatcc tccttcgctg cccaggtttc ggagaggacg gccgtgagga ggaggggcgc     480

gcggctcgcg cggaacgcgg atagcattgc acgacggccc cgcgcggagc gggcggcccg     540

tacatccaat atcttcccgc cgatgaataa ttcagggcgg cccgcgggga cctgcggccc     600

cgcgccgcgg agcgcctgag gccggagaag ggtcggagcg cgcgctagag agcgagccag     660

gaggccggag gacacgcggg cgccgggccg cgtggggcgc gcgcgcgtgt gagcgcatag     720

agaccgcggc ccggcagggc tgcgcacggg cgcggagcgt gtggggcgcg cgtgcgtact     780

tggcacgcgt gcttccgcgg gccgggggcc cccgcacgag gcgcagctgc gcgcccgagc     840

cgaccgcgcc gcgggggagg ctgcgcctcg gccgccacgc aggagctcct gccgccgcgt     900

catgcggcgg ggcggccggc ggcgcgcgcg gggacttgga ctgctgcgac tcccggacgt     960

tttcccctcc ctccaggagg ctcaattcat ttagccggag ggcaaggaaa aaaaaaaaca 1020
acacaagcaa gactcgttgg gaccaaattg gtttgcgccg cggggaccgg attccacggc 1080
ggactttgag acttgtgcgg gcgctcgttt gctgaggagt agcagtgcaa acccgccctg 1140
ccgaggtggc ccaggggtcg cctccaccgc gtcctcgatg cgctctgctg agtgagccgc 1200
gcgccctcat cctcctcgcc gcgcgccctc tgccctccag cccgcctgca aatggcttcg 1260
ttcccggaga ccgacttcca gatctgcctg ctgtgcaagg agatgtgcgg ctcgcccgcg 1320
ccgctctcct ccaactcgtc cgcctcgtcg tcgtcctcgc agacttccac gtcgtcggcg 1380
ggcggcggcg gccccggggc ggcggcgcgg cgtcttcacg tcttgccctg cctgcacgcc 1440
ttctgccggc cgtgcctcga ggcgcaccgg ctgcccgctc ccggaggcgc gggccccgcc 1500
gaggcgctga agctgcgctg ccccgtatgt gaccagaagg tggtcctggc cgaggcggcg 1560
ggcatggacg cgctgccatc ctccgccttc ctgctcagca acctgctgga cgccgtggtg 1620
gccaccgccg aagagccgcc gcccaagaac gggcgcgcgg gcggcggccc cgggggcgcg 1680
ggcggccaca gcaaccaccg gcaccatgcg caccacccgg cccagcgcgc cgccgctcca 1740
gcgccgcagc cgccgcccgg cccggccgcc tcccccgggt cgctgctgat gcgccggccg 1800
cacggctgca gctcctgcga cgagggcaac gccgcctcgt cgcgctgcct cgactgccag 1860
gaacacctgt gcgacaactg tgtccgcgcg caccagcgcg tccgcctcac caaggaccat 1920
tacatcgagc gcggccctcc ggggcctgcg gccgcctccg cggcacagca gctggggctc 1980
gggccgccct tcgcgggcgc gcccttctcc attctctcgg tgttcccgga gcgcctcggc 2040
ttctgccagc accacgacga tgaggtcctg catctctact gtgacacctg ctctgtcccc 2100
atctgcagag agtgcacgct gggccgccac ggtggccaca gcttcgccta cctccaggat 2160
gccctgcagg actctcgggc actcaccatc cagctgctgg ctgatgccca gcagggccgc 2220
caggctctcc agctgagcat cgagcaggcc cagactgtgg cagaacaggt ggagatgaag 2280
gcgaaggtgg tccagtctga ggtcaaggcc gtcacagctc gccacaagaa ggccctggag 2340
gatcgggagt gtgagctgtt gtggaaggtg gagaagatcc ggcaggtgaa ggcgaagtct 2400
ctgttcctgc aggtggagaa gctgcggcag agtctcagca agctggagag caccatcagc 2460
gccgtgcagc aggtcctgga agagggccgc gccctggaca tcctgctggc ccgagaccgg 2520
atgctggccc aggtgcagga gctcaagacc atccgaggcc tcctgcagcc ccaagaagat 2580
gaccgcatta tgttcacgcc ccctgaccag gccctgtacc tcgccctcaa gtctattggc 2640
tttgtcagca gcggggcctt cgccccgctc acgaaggcta caggagatgg catcaagcgt 2700
gcccttcagg ggaaggtggc ctctttcact gtcatgggct acgaccacga tggtgagccc 2760
cgccattcgg gaggtgacct gatgtcagtt gtggtcctcg gccccgacgg aaacctgttt 2820
ggtgcagaag tgagcgacca gcagaatggt acttacatag tgagctacag gccccagctt 2880
gagggtgagc acttggtgtc ggtgacattg tacaatcagc atatagagaa cagccccttc 2940
aaggtcgtgg tcaagtcggg ccgcagctac gtgggcatcg ggctccccgg gctgagcttc 3000

ggcagtgaag gtgacgggga gggcaagctc tgcaggccct ggggcgtgag tgttgataag 3060
gagggtttca tcatagtggc tgaccgaagc aataacagaa tccaggtgtt taagccctgt 3120
ggttccttcc accacaagtt tggtaccctt gggtctcgtc ctgggcagtt tgaccgaccg 3180
gctggggtgg cttgtgacgc ctcccgcagg atcatagtgg ccgacaaaga caatcatcgc 3240
attcagatct tcaccttcga gggccagttc cttcttaagt ttggtgagaa aggcaccaag 3300
aacgggcagt ttaactaccc gtgggatgta gcagtgaatt ctgagggcaa gatcctcgtt 3360
tcagacaccc ggaatcatcg catccagttg ttcggaccgg acggggtctt cctgaacaag 3420
tacggcttcg agggatctct ctggaagcac ttcgactctc cacggggcgt ggctttcaac 3480
aatgaaggtc acctggtagt caccgacttc aacaaccacc ggcttctggt tattcaccct 3540
gattgccaat ctgcccgctt cttgggttcg gagggctcgg gcaatgggca gttcctgcga 3600
ccacagggtg tggctgtgga ccaggaaggg agaatcatcg tagccgattc cagaaatcac 3660
cgcgtgcaga tgtttgaggc caatggcagc ttcctgtgca agtttggtgc tcaagggagt 3720
ggcttcgggc agatggaccg cccctcaggt atcgcggtca ccccggatgg attgatcgtc 3780
gtggtggact ttggcaacaa tcgaatcctc atcttctaat tgtgtcttct ggggttttct 3840
gtgtttaggg tgtgcatgtg tgtgtctctc atttttgaat ttcaaagaag aaatcgtctc 3900
agggatattt ctttttcttt ttccccccct ttatttttat tatttttttt tttttaagaa 3960
caaaagtaca acattgccta agtcctacct cagcttaaat tttttttttt tttttttttt 4020
ttacagatga atgtaattct cctctgtgca gggcttgagc ctgtgaagtg ataatttcta 4080
tctacctcaa ctctttgcat ttccctctct ggcaagcctc ttggctcaca tcctcttccc 4140
tcctgaaggc ctaggaggca caggacagcg gtcctgcaac cttgagggca ctggaagcat 4200
atggtggggt gcatgcattt tgtagattga gccaaggaaa cccaaaaact actaagtaaa 4260
aaagagaagt ataagatgtt ggaaagatag gatttaaaat tcataattgt agtgtatttg 4320
tgttcttgag aatactgtgt ttatgtgggg ttaggttgtg tgtggtttgc tcttttcttt 4380
tttatcgatg caacagggct tttctctgtt tacctcagtg tgtctgacat ctcatcccta 4440
ggagatatgg ctgtggcttg ctagcctaga aaagcagacc attcatattt tcgggtgtct 4500
catctttgta tctggtaaag aagggtgggt gtgtgggagt gtgtgtgtgc acacgcgtgc 4560
atgtttgtgt ttcaaagcca tctttgcttg aggagaaaag ctggacttga cacaagacat 4620
cccaagcaaa tttcttccca gtttgctaag aaactcgatt tagctcagga ctctaggttg 4680
aaagtgttcg caaaggttac gtgtctgtgt ttttgttgtt tgttataaag acttaggtat 4740
aagaaaataa gttaaccttg ttctcagaga gagaggggct tttcagatgt ggaccagggt 4800
gccacagaag tggggggtgc tggagaagtg aggcaccccc acatcaggga ctgttcagga 4860
gggcggggga cctcgtctct atgtgtgagg gtgtggtagg caaaggata gctttgattt 4920
gagaagcaca atgttttttc ctgctttgga agttcaggga atgtagaaga ttgtctgact 4980
ggtgggagca cctcactccc taacccttgg ctcaggtctg tttctcagga accttcccta 5040

agtctgagtg gaggcagacc tcaaggttaa cacaaagccc cgttcctccc ccaaaggcct 5100

ttgaatgtaa aggatttaca aaccaaacca cccaacagga agtgtaggac acagtatatg 5160

tcttttttttt tttttaattt tatttt 5186

<210> 5
<211> 832
<212> PRT
<213> Drosophila melanogaster

<400> 5

Met Met Glu Leu Leu Ser Asn Asn Ser Val Pro Gln Gln Met Ala Ser
1               5                   10                  15

Ser Asn Ala Pro Ser Ala Asn Asn Val Ala His Ser Ser Thr Ala Asn
            20                  25                  30

Gly Ser Gly Gly Gly Ser Val Ser Ser Asn Ala Ser Asn Ser Ser Glu
        35                  40                  45

Arg Leu Leu Ala Gly Ile Leu Glu Ser Phe Pro Ala Trp Asp Leu Asn
    50                  55                  60

Val Gly Leu Leu Pro Asn Val Gly Gln Ser Ser Pro Pro Arg Ala Asp
65                  70                  75                  80

Phe Phe Ile Asn Asn Phe Leu Gly Gly Leu Asp Thr His Gly Asp Phe
                85                  90                  95

Ser Ile Gly Pro Ile Gly Ser Gly Ala Arg Ser Asn Pro Lys Met Ser
            100                 105                 110

Pro Glu Ser Ser Asn Asn Ser Ser Ile Ser Cys Gly Trp Cys Glu Val
        115                 120                 125

Ser Ala Ser Ile Arg Cys Leu Glu Cys Asn Glu Phe Met Cys Asn Asp
    130                 135                 140

Cys Leu Arg Glu His Arg Asn Ser Pro Leu Ser Ser Asn His Ser Ile
145                 150                 155                 160

Val Ser Leu Pro Thr Pro Ile Gly Ala Ser Pro Thr Gly Gly Ser Ser
                165                 170                 175

Val Asn Ala Gln Thr Pro Pro Ser Gly Asn Phe Ile Cys Asp Ile His
            180                 185                 190

Asn Glu Met Leu Arg Tyr Val Cys Asp Tyr Cys Arg Lys Leu Val Cys
        195                 200                 205

Gln Cys Cys Thr Leu His Glu His Lys Glu His Ser Tyr Ala Ser Ile

210                 215                 220

Gln Ser Phe Met Val Gly Ser Lys Glu Lys Leu Glu Gly Ala Ile Glu
225                 230                 235                 240

Ser Ser Gln Val Gly Thr Arg Cys Ile Lys Ser Ser Ile Asp Lys Ala
                245                 250                 255

Leu Ala Phe Ile Arg Leu Ile Glu Arg Asn Cys Ser Glu Leu Ser Asp
            260                 265                 270

Asn Ile Arg Lys Ala Phe Arg Gln Phe Ile Ile Ala Ile Glu Asp Arg
        275                 280                 285

Glu Arg Phe Leu Leu Asp Phe Val Glu Lys Leu Arg Gln Arg Arg Leu
    290                 295                 300

Ala Ile Leu His Asp Gln Met Ala Gly Leu Lys Ser Ala Leu Ala Gly
305                 310                 315                 320

Leu Ser Glu Thr Ser Asp Met Leu Ser Lys Val Ala Asp Asn Ala Cys
                325                 330                 335

Asn Met Asp Gln Ile Glu Ile Ala Met Lys Leu Thr Asn Gly Gln Arg
            340                 345                 350

Gln Met Glu Gln Phe Ala Gly Ile Tyr Lys Asp Leu Gln Pro Lys Gln
        355                 360                 365

Glu Val Phe Ala Phe Ala Pro Pro Asp Tyr Ser Leu Leu Gln Asp Ile
    370                 375                 380

Arg Asn Gln Gly Gly Val Ile Leu Val Asp Asp Lys Asn Leu Pro Ile
385                 390                 395                 400

Val Ser Ser Ser Asn Gly Ile Val Pro Ser Val Ser Ser Val Asn Ala
                405                 410                 415

Val Ala Ala Ala Ser Val Gly Val Val Gly Gly Val Ala Gly Val Val
            420                 425                 430

Gly Gly Val Gly Val Ser Asn Gly Leu Asp Leu Ala Phe Gly Met Asn
        435                 440                 445

Met Pro Asn Asn Pro Leu Ser Val Ala Ser Ser Ser Val Arg Arg Pro
    450                 455                 460

Leu Leu Arg Asp Asn Ser Phe Arg Ile Pro Ser Pro Ile Met Gln Pro
465                 470                 475                 480

Arg Gly Gly Ser Ala Cys Gly Met Ser Ser Gly Met Ser Ser Ala Ala

                485                 490                 495

Leu Asp Trp Glu Leu Asn Gly Leu Arg Ser Ser Pro Gly Leu His Phe
            500                 505                 510

Ser Ala Pro Arg Thr Thr Gln Ala Ile Pro Gly Cys Met Asp Leu Val
        515                 520                 525

Lys Val Arg Asn Ser Asn Ala Leu Ser Leu Ser Phe Ala Thr Glu Gly
    530                 535                 540

His Glu Asp Gly Gln Val Ser Arg Pro Trp Gly Leu Cys Val Asp Lys
545                 550                 555                 560

Met Gly His Val Leu Val Ser Asp Arg Arg Asn Asn Arg Val Gln Val
                565                 570                 575

Phe Asn Pro Asp Gly Ser Leu Lys Phe Lys Phe Gly Arg Lys Gly Val
            580                 585                 590

Gly Asn Gly Glu Phe Asp Leu Pro Ala Gly Ile Cys Val Asp Val Asp
        595                 600                 605

Asn Arg Ile Ile Val Val Asp Lys Asp Asn His Arg Val Gln Ile Phe
    610                 615                 620

Thr Ala Ser Gly Val Phe Leu Leu Lys Phe Gly Ser Tyr Gly Lys Glu
625                 630                 635                 640

Tyr Gly Gln Phe Gln Tyr Pro Trp Asp Val Ala Val Asn Ser Arg Arg
                645                 650                 655

Gln Ile Val Val Thr Asp Ser Arg Asn His Arg Ile Gln Gln Phe Asp
            660                 665                 670

Ser Glu Gly Arg Phe Ile Arg Gln Ile Val Phe Asp Asn His Gly Gln
        675                 680                 685

Thr Lys Gly Ile Ala Ser Pro Arg Gly Val Cys Tyr Thr Pro Thr Gly
    690                 695                 700

Asn Ile Ile Val Ser Asp Phe Asp Asn His Cys Leu Tyr Leu Ile Asp
705                 710                 715                 720

Pro Asp Ile Asn Asp Ile Leu Ser Val Lys Gly His Glu Gly Ser Gly
                725                 730                 735

Phe His Glu Phe Asn Arg Pro Ser Gly Leu Cys Cys Asp Asp Glu Gly
            740                 745                 750

Arg Ile Ile Val Ala Asp Ser Lys Asn Gln Arg Ile Leu Val Phe Asn

755                 760                 765

Gln Asn Leu Asp Phe Met Trp Asp Ile Glu Val Arg Pro Ser Ile Asn
    770                 775                 780

Pro Leu Met Pro Pro Thr Leu Asp Glu Lys Asp Arg Thr Cys Asp Val
785                 790                 795                 800

Ala Ile Met Pro Asp Gly Arg Ile Val Phe Leu Ile Glu Leu Ser Pro
                805                 810                 815

Asp Ser Lys Glu Gly Ser Asn Pro Tyr Lys Arg Phe Val His Val Phe
            820                 825                 830

<210>  6
<211>  832
<212>  PRT
<213>  Drosophila melanogaster

<400>  6

Met Met Glu Leu Leu Ser Asn Asn Ser Val Pro Gln Gln Met Ala Ser
1               5                   10                  15

Ser Asn Ala Pro Ser Ala Asn Asn Val Ala His Ser Ser Thr Ala Asn
            20                  25                  30

Gly Ser Gly Gly Gly Ser Val Ser Ser Asn Ala Ser Asn Ser Ser Glu
        35                  40                  45

Arg Leu Leu Ala Gly Ile Leu Glu Ser Phe Pro Ala Trp Asp Leu Asn
    50                  55                  60

Val Gly Leu Leu Pro Asn Val Gly Gln Ser Ser Pro Pro Arg Ala Asp
65                  70                  75                  80

Phe Phe Ile Asn Asn Phe Leu Gly Gly Leu Asp Thr His Gly Asp Phe
                85                  90                  95

Ser Ile Gly Pro Ile Gly Ser Gly Ala Arg Ser Asn Pro Lys Met Ser
            100                 105                 110

Pro Glu Ser Ser Asn Asn Ser Ser Ile Ser Cys Gly Trp Cys Glu Val
        115                 120                 125

Ser Ala Ser Ile Arg Cys Leu Glu Cys Asn Glu Phe Met Cys Asn Asp
    130                 135                 140

Cys Leu Arg Glu His Arg Asn Ser Pro Leu Ser Ser Asn His Ser Ile
145                 150                 155                 160

Val Ser Leu Pro Thr Pro Ile Gly Ala Ser Pro Thr Gly Gly Ser Ser
                165                 170                 175

Val Asn Ala Gln Thr Pro Pro Ser Gly Asn Phe Ile Cys Asp Ile His
            180                 185                 190

Asn Glu Met Leu Arg Tyr Val Cys Asp Tyr Cys Arg Lys Leu Val Cys
        195                 200                 205

Gln Cys Cys Thr Leu His Glu His Lys Glu His Ser Tyr Ala Ser Ile
    210                 215                 220

Gln Ser Phe Met Val Gly Ser Lys Glu Lys Leu Glu Gly Ala Ile Glu
225                 230                 235                 240

Ser Ser Gln Val Gly Thr Arg Cys Ile Lys Ser Ser Ile Asp Lys Ala
                245                 250                 255

Leu Ala Phe Ile Arg Leu Ile Glu Arg Asn Cys Ser Glu Leu Ser Asp
            260                 265                 270

Asn Ile Arg Lys Ala Phe Arg Gln Phe Ile Ile Ala Ile Glu Asp Arg
        275                 280                 285

Glu Arg Phe Leu Leu Asp Phe Val Glu Lys Leu Arg Gln Arg Arg Leu
    290                 295                 300

Ala Ile Leu His Asp Gln Met Ala Gly Leu Lys Ser Ala Leu Ala Gly
305                 310                 315                 320

Leu Ser Glu Thr Ser Asp Met Leu Ser Lys Val Ala Asp Asn Ala Cys
                325                 330                 335

Asn Met Asp Gln Ile Glu Ile Ala Met Lys Leu Thr Asn Gly Gln Arg
            340                 345                 350

Gln Met Glu Gln Phe Ala Gly Ile Tyr Lys Asp Leu Gln Pro Lys Gln
        355                 360                 365

Glu Val Phe Ala Phe Ala Pro Pro Asp Tyr Ser Leu Leu Gln Asp Ile
    370                 375                 380

Arg Asn Gln Gly Gly Val Ile Leu Val Asp Asp Lys Asn Leu Pro Ile
385                 390                 395                 400

Val Ser Ser Ser Asn Gly Ile Val Pro Ser Val Ser Ser Val Asn Ala
                405                 410                 415

Val Ala Ala Ala Ser Val Gly Val Val Gly Gly Val Ala Gly Val Val
            420                 425                 430

Gly Gly Val Gly Val Ser Asn Gly Leu Asp Leu Ala Phe Gly Met Asn
        435                 440                 445

Met Pro Asn Asn Pro Leu Ser Val Ala Ser Ser Ser Val Arg Arg Pro
    450                 455                 460

Leu Leu Arg Asp Asn Ser Phe Arg Ile Pro Ser Pro Ile Met Gln Pro
465                 470                 475                 480

Arg Gly Gly Ser Ala Cys Gly Met Ser Ser Gly Met Ser Ser Ala Ala
                485                 490                 495

Leu Asp Trp Glu Leu Asn Gly Leu Arg Ser Ser Pro Gly Leu His Phe
            500                 505                 510

Ser Ala Pro Arg Thr Thr Gln Ala Ile Pro Gly Cys Met Asp Leu Val
        515                 520                 525

Lys Val Arg Asn Ser Asn Ala Leu Ser Leu Ser Phe Ala Thr Glu Gly
    530                 535                 540

His Glu Asp Gly Gln Val Ser Arg Pro Trp Gly Leu Cys Val Asp Lys
545                 550                 555                 560

Met Gly His Val Leu Val Ser Asp Arg Arg Asn Asn Arg Val Gln Val
                565                 570                 575

Phe Asn Pro Asp Gly Ser Leu Lys Phe Lys Phe Gly Arg Lys Gly Val
            580                 585                 590

Gly Asn Gly Glu Phe Asp Leu Pro Ala Gly Ile Cys Val Asp Val Asp
        595                 600                 605

Asn Arg Ile Ile Val Val Asp Lys Asp Asn His Arg Val Gln Ile Phe
    610                 615                 620

Thr Ala Ser Gly Val Phe Leu Leu Lys Phe Gly Ser Tyr Gly Lys Glu
625                 630                 635                 640

Tyr Gly Gln Phe Gln Tyr Pro Trp Asp Val Ala Val Asn Ser Arg Arg
                645                 650                 655

Gln Ile Val Val Thr Asp Ser Arg Asn His Arg Ile Gln Gln Phe Asp
            660                 665                 670

Ser Glu Gly Arg Phe Ile Arg Gln Ile Val Phe Asp Asn His Gly Gln
        675                 680                 685

Thr Lys Gly Ile Ala Ser Pro Arg Gly Val Cys Tyr Thr Pro Thr Gly
    690                 695                 700

Asn Ile Ile Val Ser Asp Phe Asp Asn His Cys Leu Tyr Leu Ile Asp
705                 710                 715                 720

Pro Asp Ile Asn Asp Ile Leu Ser Val Lys Gly His Glu Gly Ser Gly
                725                 730                 735

Phe His Glu Phe Asn Arg Pro Ser Gly Leu Cys Cys Asp Asp Glu Gly
            740                 745                 750

Arg Ile Ile Val Ala Asp Ser Lys Asn Gln Arg Ile Leu Val Phe Asn
        755                 760                 765

Gln Asn Leu Asp Phe Met Trp Asp Ile Glu Val Arg Pro Ser Ile Asn
    770                 775                 780

Pro Leu Met Pro Pro Thr Leu Asp Glu Lys Asp Arg Thr Cys Asp Val
785                 790                 795                 800

Ala Ile Met Pro Asp Gly Arg Ile Val Phe Leu Ile Glu Leu Ser Pro
                805                 810                 815

Asp Ser Lys Glu Gly Ser Asn Pro Tyr Lys Arg Phe Val His Val Phe
            820                 825                 830

<210>  7
<211>  832
<212>  PRT
<213>  Drosophila melanogaster

<400>  7

Met Met Glu Leu Leu Ser Asn Asn Ser Val Pro Gln Gln Met Ala Ser
1               5                   10                  15

Ser Asn Ala Pro Ser Ala Asn Asn Val Ala His Ser Ser Thr Ala Asn
            20                  25                  30

Gly Ser Gly Gly Gly Ser Val Ser Ser Asn Ala Ser Asn Ser Ser Glu
        35                  40                  45

Arg Leu Leu Ala Gly Ile Leu Glu Ser Phe Pro Ala Trp Asp Leu Asn
    50                  55                  60

Val Gly Leu Leu Pro Asn Val Gly Gln Ser Ser Pro Pro Arg Ala Asp
65                  70                  75                  80

Phe Phe Ile Asn Asn Phe Leu Gly Gly Leu Asp Thr His Gly Asp Phe
                85                  90                  95

Ser Ile Gly Pro Ile Gly Ser Gly Ala Arg Ser Asn Pro Lys Met Ser
            100                 105                 110

Pro Glu Ser Ser Asn Asn Ser Ser Ile Ser Cys Gly Trp Cys Glu Val
        115                 120                 125

Ser Ala Ser Ile Arg Cys Leu Glu Cys Asn Glu Phe Met Cys Asn Asp
    130                 135                 140

Cys Leu Arg Glu His Arg Asn Ser Pro Leu Ser Ser Asn His Ser Ile
145                 150                 155                 160

Val Ser Leu Pro Thr Pro Ile Gly Ala Ser Pro Thr Gly Gly Ser Ser
                165                 170                 175

Val Asn Ala Gln Thr Pro Pro Ser Gly Asn Phe Ile Cys Asp Ile His
            180                 185                 190

Asn Glu Met Leu Arg Tyr Val Cys Asp Tyr Cys Arg Lys Leu Val Cys
        195                 200                 205

Gln Cys Cys Thr Leu His Glu His Lys Glu His Ser Tyr Ala Ser Ile
    210                 215                 220

Gln Ser Phe Met Val Gly Ser Lys Glu Lys Leu Glu Gly Ala Ile Glu
225                 230                 235                 240

Ser Ser Gln Val Gly Thr Arg Cys Ile Lys Ser Ser Ile Asp Lys Ala
                245                 250                 255

Leu Ala Phe Ile Arg Leu Ile Glu Arg Asn Cys Ser Glu Leu Ser Asp
            260                 265                 270

Asn Ile Arg Lys Ala Phe Arg Gln Phe Ile Ile Ala Ile Glu Asp Arg
        275                 280                 285

Glu Arg Phe Leu Leu Asp Phe Val Glu Lys Leu Arg Gln Arg Arg Leu
    290                 295                 300

Ala Ile Leu His Asp Gln Met Ala Gly Leu Lys Ser Ala Leu Ala Gly
305                 310                 315                 320

Leu Ser Glu Thr Ser Asp Met Leu Ser Lys Val Ala Asp Asn Ala Cys
                325                 330                 335

Asn Met Asp Gln Ile Glu Ile Ala Met Lys Leu Thr Asn Gly Gln Arg
            340                 345                 350

Gln Met Glu Gln Phe Ala Gly Ile Tyr Lys Asp Leu Gln Pro Lys Gln
        355                 360                 365

Glu Val Phe Ala Phe Ala Pro Pro Asp Tyr Ser Leu Leu Gln Asp Ile
    370                 375                 380

Arg Asn Gln Gly Gly Val Ile Leu Val Asp Asp Lys Asn Leu Pro Ile
385                 390                 395                 400

Val Ser Ser Ser Asn Gly Ile Val Pro Ser Val Ser Ser Val Asn Ala
                405                 410                 415

Val Ala Ala Ala Ser Val Gly Val Val Gly Gly Val Ala Gly Val Val
            420                 425                 430

Gly Gly Val Gly Val Ser Asn Gly Leu Asp Leu Ala Phe Gly Met Asn
        435                 440                 445

Met Pro Asn Asn Pro Leu Ser Val Ala Ser Ser Ser Val Arg Arg Pro
    450                 455                 460

Leu Leu Arg Asp Asn Ser Phe Arg Ile Pro Ser Pro Ile Met Gln Pro
465                 470                 475                 480

Arg Gly Gly Ser Ala Cys Gly Met Ser Ser Gly Met Ser Ser Ala Ala
                485                 490                 495

Leu Asp Trp Glu Leu Asn Gly Leu Arg Ser Ser Pro Gly Leu His Phe
            500                 505                 510

Ser Ala Pro Arg Thr Thr Gln Ala Ile Pro Gly Cys Met Asp Leu Val
        515                 520                 525

Lys Val Arg Asn Ser Asn Ala Leu Ser Leu Ser Phe Ala Thr Glu Gly
    530                 535                 540

His Glu Asp Gly Gln Val Ser Arg Pro Trp Gly Leu Cys Val Asp Lys
545                 550                 555                 560

Met Gly His Val Leu Val Ser Asp Arg Arg Asn Asn Arg Val Gln Val
                565                 570                 575

Phe Asn Pro Asp Gly Ser Leu Lys Phe Lys Phe Gly Arg Lys Gly Val
            580                 585                 590

Gly Asn Gly Glu Phe Asp Leu Pro Ala Gly Ile Cys Val Asp Val Asp
        595                 600                 605

Asn Arg Ile Ile Val Val Asp Lys Asp Asn His Arg Val Gln Ile Phe
    610                 615                 620

Thr Ala Ser Gly Val Phe Leu Leu Lys Phe Gly Ser Tyr Gly Lys Glu
625                 630                 635                 640

Tyr Gly Gln Phe Gln Tyr Pro Trp Asp Val Ala Val Asn Ser Arg Arg
                645                 650                 655

Gln Ile Val Val Thr Asp Ser Arg Asn His Arg Ile Gln Gln Phe Asp
            660                 665                 670

Ser Glu Gly Arg Phe Ile Arg Gln Ile Val Phe Asp Asn His Gly Gln
        675                 680                 685

Thr Lys Gly Ile Ala Ser Pro Arg Gly Val Cys Tyr Thr Pro Thr Gly
    690                 695                 700

Asn Ile Ile Val Ser Asp Phe Asp Asn His Cys Leu Tyr Leu Ile Asp
705                 710                 715                 720

Pro Asp Ile Asn Asp Ile Leu Ser Val Lys Gly His Glu Gly Ser Gly
                725                 730                 735

Phe His Glu Phe Asn Arg Pro Ser Gly Leu Cys Cys Asp Asp Glu Gly
            740                 745                 750

Arg Ile Ile Val Ala Asp Ser Lys Asn Gln Arg Ile Leu Val Phe Asn
        755                 760                 765

Gln Asn Leu Asp Phe Met Trp Asp Ile Glu Val Arg Pro Ser Ile Asn
    770                 775                 780

Pro Leu Met Pro Pro Thr Leu Asp Glu Lys Asp Arg Thr Cys Asp Val
785                 790                 795                 800

Ala Ile Met Pro Asp Gly Arg Ile Val Phe Leu Ile Glu Leu Ser Pro
                805                 810                 815

Asp Ser Lys Glu Gly Ser Asn Pro Tyr Lys Arg Phe Val His Val Phe
            820                 825                 830

<210>  8
<211>  4516
<212>  DNA
<213>  Drosophila melanogaster

<400>  8
cagcggcgag cgcatgaacg tgagttttgc agtttgatgg ttaaatgcgt gcgtgaaggt      60

tggagtgaag attttgagcc gaattcgaat cctagtcccc gccgcaataa gtgggtcaga     120

attggagccg ttcgattggg ccaaacaaaa agctgggcgc caccgcagac tgcagctaaa     180

acacgccgcc aaaaaggaaa agtgcacgag ggtggaacca agggtaaata agattcgccc     240

gaaaacgctt ggaaaatgta aacaatctgt aagcacagcg acgataagcg attttcaaag     300

agtttgccag ctttttacat cgatcaaaca ctcgcataat aaacgaataa gaactgacga     360

ttaaatcgaa ttgaagtgga gtgacgacta tgggataccc taggaccgcg tagagtgaat     420

gaatttgaca acggaaaagc gccactccat tgactgctgc cttgtgctat agaaacgtga     480

tctcaccact cttatgatgg aacttttgtc aaacaattcg gttccgcaac aaatggccag     540

cagcaatgcg ccgagcgcca acaatgtggc ccactcctca acggccaacg gaagtggagg     600

cggcagtgtg agcagcaatg ccagcaactc ctccgagcga ctgctggcgg gcatcctgga     660

gagctttccc gcctgggacc tcaacgtggg actactgccc aacgtgggcc agagctcacc 720
accacgcgca gacttcttta tcaataactt cctgggcggc ctggacacgc atggcgactt 780
cagcattgga cccattggca gcggtgcccg cagcaatccg aagatgtcgc cagagtcatc 840
taacaactcg agcatcagct gcggttggtg cgaggttagt gcttcaatac gctgcctgga 900
gtgcaacgag ttcatgtgca acgactgcct aagggagcat cgcaacagtc cgctgtcctc 960
caaccactcg atcgtgtcct tgcccacgcc cattggagcc tcgcccacgg gtggcagctc 1020
ggtaaatgca cagactccgc ccagcggcaa ctttatctgc gacatacaca acgagatgtt 1080
gcgctacgta tgtgactact gccggaaatt ggtgtgtcag tgctgcacac tgcacgagca 1140
caaggagcac agctacgcgt ccatccaaag ctttatggtg ggctcgaagg agaagctgga 1200
gggcgccatt gagagcagcc aggtgggcac gcgctgcatt aagagcagca ttgacaaagc 1260
gttggccttc atccggctta tcgagcgcaa ttgcagcgag ctgagcgata atatacgcaa 1320
ggcattccgt cagtttatca ttgccatcga ggaccgcgag cgtttcctcc tggactttgt 1380
ggaaaagctc cgccagcgtc gtctggccat cctacacgat cagatggcag gcttaaagtc 1440
tgctctcgcc ggactctccg aaacgtccga tatgcttagc aaggtggcgg acaatgcctg 1500
caacatggac cagattgaaa ttgccatgaa gttgaccaat gggcagaggc agatggagca 1560
gtttgcgggc atatataagg acctgcagcc aaaacaggaa gtctttgcct tcgcaccacc 1620
agattacagc ctgctacagg atatccgcaa ccagggtggc gttatcctgg tggacgacaa 1680
gaacttgccc atcgtctcta gcagcaacgg aattgtgccg agcgtctcca gtgttaatgc 1740
cgtggccgcg gcctccgtgg gagttgtggg aggcgttgct ggagtagtcg gaggcgttgg 1800
cgtgtcaaac ggcctggact tggccttcgg catgaacatg cccaacaatc cgctgagtgt 1860
tgcttcctca agtgtgcgac gtcccctgtt gcgagacaat agcttccgga ttccatcgcc 1920
cataatgcaa ccacgcggag gaagtgcctg tgggatgtcc agtgggatgt ccagcgctgc 1980
tcttgattgg gaactcaatg gactgcgcag ctcgccagga ttacacttca gtgcgccgcg 2040
aaccacgcag gccattccgg gatgcatgga tctggtcaag gtgcgtaact cgaatgctct 2100
ctcgctatcc ttcgccaccg agggccacga ggacggacag gtgagccggc catggggtct 2160
atgcgttgat aaaatgggcc acgttctcgt atcggatcgc cgcaacaatc gcgtccaggt 2220
cttcaatccc gatggctccc taaagttcaa attcggccgt aaaggcgtgg gcaatggaga 2280
gttcgatctg cctgctggaa tctgtgttga cgttgataat cgcattattg tggtcgacaa 2340
ggacaatcac cgtgtgcaaa tctttaccgc cagcggcgtc ttcctgctca agttcggcag 2400
ctacggcaag gagtacggcc agttccagta tccgtgggat gtggccgtga actcgcgccg 2460
ccaaattgtg gttaccgact cgcgcaatca tcgcatccag cagttcgact ctgagggtcg 2520
cttcatccgc cagatagtgt tcgacaacca tgggcaaacc aaaggaatcg cctcgccacg 2580
aggcgtttgc tacacgccga cgggcaacat catagtgtct gactttgaca accactgcct 2640
ctacctgatt gatccagaca tcaacgacat tctttccgtg aaggacatga agggctcggg 2700

cttccatgaa tttaatcgac cctccggcct gtgctgtgac gacgagggcc gcattatagt 2760
ggccgattca aagaatcaac gcatcctggt cttcaatcag aacctggact ttatgtggga 2820
tatcgaggtg cgaccctcca tcaatccctt gatgccgccc acgctggacg agaaagatcg 2880
cacttgcgat gtggccataa tgccagacgg tcgcattgtg ttcctcattg aactgtcgcc 2940
agactccaag gaagggtcaa acccttacaa gcggtttgtg cacgtattct aaataaggcc 3000
acgagtgccg atcttttttt ttagaccatt tacaagagcg aaaccttagc gtagagtttc 3060
aaataacatg acgattatga ggaggtgccg aaaccgattt aataagaaaa aatctgaaat 3120
catgaaagtt agcgaaaatt tttagcttaa gtgctacaac gattacctcg atattgctta 3180
agagttatgg ttacgatttg gtaatttaca atacaatacg aattacacat atgtatctat 3240
ggatatctat atctatacgc atatgcatat atctagcgaa tatacaagcc caaccaacta 3300
gaactataac taaaacaaaa acaaaaaaca aaaactaaag cgaaaggcag gcggttgagt 3360
aggatttagg agatatatat aaacgtaggg tccgtgagca ttcagtagag ttaccgaacc 3420
tagtaaaaga taatgcaaag agggaactca accatttact agatgaaaat attattacct 3480
gttagttaga ctgaaaggca gtttgctagc tgatagaagg aggtacgaac caggtcaggt 3540
ggcgattgtg ttttttttta cgggtttgct tagcggaatg aggaaactac attcgacaag 3600
agttactaaa catgatttgc agaaacactg tatggtatgg tattaatgtt tgtattatat 3660
ttatagttcg tttgttcagt tctgtttttc gtttatttta aaattacttt ttttttggga 3720
catacatatg tattttaatg atttccttat aattgagact gagcatttga caacacgaac 3780
tttgcgtgta cggaagcaaa gcgaaacaaa acaaagaaac aaacaaaaaa caagaaaata 3840
ttgatggaac ccgctgactt ttcgttgaac tttatacaaa gactgattaa ctaaaccgca 3900
atttcggagc caactaaaga accatatatg gtgaggccaa aataagtgaa agagcaacac 3960
actcaaattg caatgacatt gataacgtaa agtaacttaa catagcttaa catacatact 4020
ttcgcgcata tagagcgata cagatatcat atagatccga tcatccagtc acgaatcatc 4080
atgggcatag ctgaaaccga gtttaactga cattcatcga catatttacc catccatcca 4140
tccatccatc cactcactca taccgatcat gcatcacagg gctttgtttt tcgtttgtcg 4200
ttactttgta attattgtga aaggatttag ccacaggata cagattcacg gcaggttgaa 4260
aggaggagga ggaggaggga ggggttagct aaaattagtg ggacattttg ggaagtaaac 4320
gaaacagacc tgaaacaaaa taacgaagaa gttataattt ttgactcaat gtgttcatta 4380
gcatttagta gacttagcgg gcgtggcata gtgaaaacgt atggaaaatc cagagcaata 4440
taagggcgta ttgaatacac atttaggtga catgttcctc gcaaaagaaa caaataaaac 4500
aaaacaaaag aaaaca 4516

<210> 9
<211> 4591
<212> DNA
<213> Drosophila melanogaster

<400> 9
cagcggcgag cgcatgaacg tgagttttgc agtttgatgg ttaaatgcgt gcgtgaaggt 60
tggagtgaag attttgagcc gaattcgaat cctagtcccc gccgcaataa gtgggtcaga 120
attggagccg ttcgattggg ccaaacaaaa agctgggcgc caccgcagac tgcagctaaa 180
acacgccgcc aaagtaattg gaagaagaag caggcgcaca ataggaataa ttgttcatgg 240
attttttcga tttctcgttc gcttgcagaa ggaaaagtgc acgagggtgg aaccaagggt 300
aaataagatt cgcccgaaaa cgcttggaaa atgtaaacaa tctgtaagca cagcgacgat 360
aagcgatttt caaagagttt gccagctttt tacatcgatc aaacactcgc ataataaacg 420
aataagaact gacgattaaa tcgaattgaa gtggagtgac gactatggga taccctagga 480
ccgcgtagag tgaatgaatt tgacaacgga aaagcgccac tccattgact gctgccttgt 540
gctatagaaa cgtgatctca ccactcttat gatggaactt ttgtcaaaca attcggttcc 600
gcaacaaatg gccagcagca atgcgccgag cgccaacaat gtggcccact cctcaacggc 660
caacggaagt ggaggcggca gtgtgagcag caatgccagc aactcctccg agcgactgct 720
ggcgggcatc ctggagagct ttcccgcctg ggacctcaac gtgggactac tgcccaacgt 780
gggccagagc tcaccaccac gcgcagactt ctttatcaat aacttcctgg gcggcctgga 840
cacgcatggc gacttcagca ttggacccat tggcagcggt gcccgcagca atccgaagat 900
gtcgccagag tcatctaaca actcgagcat cagctgcggt tggtgcgagg ttagtgcttc 960
aatacgctgc ctggagtgca acgagttcat gtgcaacgac tgcctaaggg agcatcgcaa 1020
cagtccgctg tcctccaacc actcgatcgt gtccttgccc acgcccattg gagcctcgcc 1080
cacgggtggc agctcggtaa atgcacagac tccgcccagc ggcaacttta tctgcgacat 1140
acacaacgag atgttgcgct acgtatgtga ctactgccgg aaattggtgt gtcagtgctg 1200
cacactgcac gagcacaagg agcacagcta cgcgtccatc caaagcttta tggtgggctc 1260
gaaggagaag ctggagggcg ccattgagag cagccaggtg ggcacgcgct gcattaagag 1320
cagcattgac aaagcgttgg ccttcatccg gcttatcgag cgcaattgca gcgagctgag 1380
cgataatata cgcaaggcat tccgtcagtt tatcattgcc atcgaggacc gcgagcgttt 1440
cctcctggac tttgtggaaa agctccgcca gcgtcgtctg gccatcctac acgatcagat 1500
ggcaggctta aagtctgctc tcgccggact ctccgaaacg tccgatatgc ttagcaaggt 1560
ggcggacaat gcctgcaaca tggaccagat tgaaattgcc atgaagttga ccaatgggca 1620
gaggcagatg gagcagtttg cgggcatata taaggacctg cagccaaaac aggaagtctt 1680
tgccttcgca ccaccagatt acagcctgct acaggatatc cgcaaccagg gtggcgttat 1740
cctggtggac gacaagaact tgcccatcgt ctctagcagc aacggaattg tgccgagcgt 1800
ctccagtgtt aatgccgtgg ccgcggcctc cgtgggagtt gtgggaggcg ttgctggagt 1860
agtcggaggc gttggcgtgt caaacggcct ggacttggcc ttcggcatga acatgcccaa 1920
caatccgctg agtgttgctt cctcaagtgt gcgacgtccc ctgttgcgag acaatagctt 1980

ccggattcca tcgcccataa tgcaaccacg cggaggaagt gcctgtggga tgtccagtgg 2040
gatgtccagc gctgctcttg attgggaact caatggactg cgcagctcgc caggattaca 2100
cttcagtgcg ccgcgaacca cgcaggccat tccgggatgc atggatctgg tcaaggtgcg 2160
taactcgaat gctctctcgc tatccttcgc caccgagggc cacgaggacg gacaggtgag 2220
ccggccatgg ggtctatgcg ttgataaaat gggccacgtt ctcgtatcgg atcgccgcaa 2280
caatcgcgtc caggtcttca atcccgatgg ctccctaaag ttcaaattcg gccgtaaagg 2340
cgtgggcaat ggagagttcg atctgcctgc tggaatctgt gttgacgttg ataatcgcat 2400
tattgtggtc gacaaggaca atcaccgtgt gcaaatcttt accgccagcg gcgtcttcct 2460
gctcaagttc ggcagctacg gcaaggagta cggccagttc cagtatccgt gggatgtggc 2520
cgtgaactcg cgccgccaaa ttgtggttac cgactcgcgc aatcatcgca tccagcagtt 2580
cgactctgag ggtcgcttca tccgccagat agtgttcgac aaccatgggc aaaccaaagg 2640
aatcgcctcg ccacgaggcg tttgctacac gccgacgggc aacatcatag tgtctgactt 2700
tgacaaccac tgcctctacc tgattgatcc agacatcaac gacattcttt ccgtgaaggg 2760
acatgagggc tcgggcttcc atgaatttaa tcgaccctcc ggcctgtgct gtgacgacga 2820
gggccgcatt atagtggccg attcaaagaa tcaacgcatc ctggtcttca atcagaacct 2880
ggactttatg tgggatatcg aggtgcgacc ctccatcaat cccttgatgc cgcccacgct 2940
ggacgagaaa gatcgcactt gcgatgtggc cataatgcca gacggtcgca ttgtgttcct 3000
cattgaactg tcgccagact ccaaggaagg gtcaaaccct tacaagcggt ttgtgcacgt 3060
attctaaata aggccacgag tgccgatctt tttttttaga ccatttacaa gagcgaaacc 3120
ttagcgtaga gtttcaaata acatgacgat tatgaggagg tgccgaaacc gatttaataa 3180
gaaaaaatct gaaatcatga aagttagcga aaatttttag cttaagtgct acaacgatta 3240
cctcgatatt gcttaagagt tatggttacg atttggtaat ttacaataca atacgaatta 3300
cacatatgta tctatggata tctatatcta tacgcatatg catatatcta gcgaatatac 3360
aagcccaacc aactagaact ataactaaaa caaaaacaaa aaacaaaaac taaagcgaaa 3420
ggcaggcggt tgagtaggat ttaggagata tatataaacg tagggtccgt gagcattcag 3480
tagagttacc gaacctagta aaagataatg caaagaggga actcaaccat ttactagatg 3540
aaaatattat tacctgttag ttagactgaa aggcagtttg ctagctgata gaaggaggta 3600
cgaaccaggt caggtggcga ttgtgttttt ttttacgggt ttgcttagcg gaatgaggaa 3660
actacattcg acaagagtta ctaaacatga tttgcagaaa cactgtatgg tatggtatta 3720
atgtttgtat tatatttata gttcgtttgt tcagttctgt tttcgtttta ttttaaaatt 3780
actttttttt tgggacatac atatgtattt taatgatttc cttataattg agactgagca 3840
tttgacaaca cgaactttgc gtgtacggaa gcaaagcgaa acaaaacaaa gaaacaaaca 3900
aaaaacaaga aaatattgat ggaacccgct gacttttcgt tgaactttat acaaagactg 3960
attaactaaa ccgcaatttc ggagccaact aaagaaccat atatggtgag gccaaaataa 4020

gtgaaagagc aacacactca aattgcaatg acattgataa cgtaaagtaa cttaacatag 4080

cttaacatac atactttcgc gcatatagag cgatacagat atcatataga tccgatcatc 4140

cagtcacgaa tcatcatggg catagctgaa accgagttta actgacattc atcgacatat 4200

ttacccatcc atccatccat ccatccactc actcataccg atcatgcatc acagggcttt 4260

gtttttcgtt tgtcgttact ttgtaattat tgtgaaagga tttagccaca ggatacagat 4320

tcacggcagg ttgaaaggag gaggaggagg agggaggggt tagctaaaat tagtgggaca 4380

ttttgggaag taaacgaaac agacctgaaa caaaataacg aagaagttat aatttttgac 4440

tcaatgtgtt cattagcatt tagtagactt agcgggcgtg gcatagtgaa aacgtatgga 4500

aaatccagag caatataagg gcgtattgaa tacacattta ggtgacatgt tcctcgcaaa 4560

agaaacaaat aaaacaaaac aaaagaaaac a 4591

<210> 10
<211> 4367
<212> DNA
<213> Drosophila melanogaster

<400> 10
agagaagaag aggaagaggc aacggcagcg gcgagcgcat gaacaaggaa aagtgcacga 60

gggtggaacc aagggtaaat aagattcgcc cgaaaacgct tggaaaatgt aaacaatctg 120

taagcacagc gacgataagc gattttcaaa gagtttgcca gctttttaca tcgatcaaac 180

actcgcataa taaacgaata agaactgacg attaaatcga attgaagtgg agtgacgact 240

atgggatacc ctaggaccgc gtagagtgaa tgaatttgac aacggaaaag cgccactcca 300

ttgactgctg ccttgtgcta tagaaacgtg atctcaccac tcttatgatg gaacttttgt 360

caaacaattc ggttccgcaa caaatggcca gcagcaatgc gccgagcgcc aacaatgtgg 420

cccactcctc aacggccaac ggaagtggag gcggcagtgt gagcagcaat gccagcaact 480

cctccgagcg actgctggcg ggcatcctgg agagctttcc cgcctgggac ctcaacgtgg 540

gactactgcc caacgtgggc cagagctcac caccacgcgc agacttcttt atcaataact 600

tcctgggcgg cctggacacg catggcgact tcagcattgg acccattggc agcggtgccc 660

gcagcaatcc gaagatgtcg ccagagtcat ctaacaactc gagcatcagc tgcggttggt 720

gcgaggttag tgcttcaata cgctgcctgg agtgcaacga gttcatgtgc aacgactgcc 780

taagggagca tcgcaacagt ccgctgtcct ccaaccactc gatcgtgtcc ttgcccacgc 840

ccattggagc ctcgcccacg ggtggcagct cggtaaatgc acagactccg cccagcggca 900

actttatctg cgacatacac aacgagatgt tgcgctacgt atgtgactac tgccggaaat 960

tggtgtgtca gtgctgcaca ctgcacgagc acaaggagca cagctacgcg tccatccaaa 1020

gctttatggt gggctcgaag gagaagctgg agggcgccat tgagagcagc caggtgggca 1080

cgcgctgcat taagagcagc attgacaaag cgttggcctt catccggctt atcgagcgca 1140

attgcagcga gctgagcgat aatatacgca aggcattccg tcagtttatc attgccatcg 1200

aggaccgcga gcgtttcctc ctggactttg tggaaaagct ccgccagcgt cgtctggcca 1260

tcctacacga tcagatggca ggcttaaagt ctgctctcgc cggactctcc gaaacgtccg 1320
atatgcttag caaggtggcg gacaatgcct gcaacatgga ccagattgaa attgccatga 1380
agttgaccaa tgggcagagg cagatggagc agtttgcggg catatataag gacctgcagc 1440
caaaacagga agtctttgcc ttcgcaccac cagattacag cctgctacag gatatccgca 1500
accagggtgg cgttatcctg gtggacgaca agaacttgcc catcgtctct agcagcaacg 1560
gaattgtgcc gagcgtctcc agtgttaatg ccgtggccgc ggcctccgtg ggagttgtgg 1620
gaggcgttgc tggagtagtc ggaggcgttg gcgtgtcaaa cggcctggac ttggccttcg 1680
gcatgaacat gcccaacaat ccgctgagtg ttgcttcctc aagtgtgcga cgtcccctgt 1740
tgcgagacaa tagcttccgg attccatcgc ccataatgca accacgcgga ggaagtgcct 1800
gtgggatgtc cagtgggatg tccagcgctg ctcttgattg ggaactcaat ggactgcgca 1860
gctcgccagg attacacttc agtgcgccgc gaaccacgca ggccattccg ggatgcatgg 1920
atctggtcaa ggtgcgtaac tcgaatgctc tctcgctatc cttcgccacc gagggccacg 1980
aggacggaca ggtgagccgg ccatggggtc tatgcgttga taaaatgggc cacgttctcg 2040
tatcggatcg ccgcaacaat cgcgtccagg tcttcaatcc cgatggctcc ctaaagttca 2100
aattcggccg taaaggcgtg ggcaatggag agttcgatct gcctgctgga atctgtgttg 2160
acgttgataa tcgcattatt gtggtcgaca aggacaatca ccgtgtgcaa atctttaccg 2220
ccagcggcgt cttcctgctc aagttcggca gctacggcaa ggagtacggc cagttccagt 2280
atccgtggga tgtggccgtg aactcgcgcc gccaaattgt ggttaccgac tcgcgcaatc 2340
atcgcatcca gcagttcgac tctgagggtc gcttcatccg ccagatagtg ttcgacaacc 2400
atgggcaaac caaaggaatc gcctcgccac gaggcgtttg ctacacgccg acgggcaaca 2460
tcatagtgtc tgactttgac aaccactgcc tctacctgat tgatccagac atcaacgaca 2520
ttctttccgt gaagggacat gagggctcgg gcttccatga atttaatcga ccctccggcc 2580
tgtgctgtga cgacgagggc cgcattatag tggccgattc aaagaatcaa cgcatcctgg 2640
tcttcaatca gaacctggac tttatgtggg atatcgaggt gcgaccctcc atcaatccct 2700
tgatgccgcc cacgctggac gagaaagatc gcacttgcga tgtggccata atgccagacg 2760
gtcgcattgt gttcctcatt gaactgtcgc cagactccaa ggaagggtca aacccttaca 2820
agcggtttgt gcacgtattc taaataaggc cacgagtgcc gatctttttt tttagaccat 2880
ttacaagagc gaaaccttag cgtagagttt caaataacat gacgattatg aggaggtgcc 2940
gaaaccgatt taataagaaa aaatctgaaa tcatgaaagt tagcgaaaat ttttagctta 3000
agtgctacaa cgattacctc gatattgctt aagagttatg gttacgattt ggtaatttac 3060
aatacaatac gaattacaca tatgtatcta tggatatcta tatctatacg catatgcata 3120
tatctagcga atatacaagc ccaaccaact agaactataa ctaaaacaaa aacaaaaaac 3180
aaaaactaaa gcgaaaggca ggcggttgag taggatttag gagatatata taaacgtagg 3240
gtccgtgagc attcagtaga gttaccgaac ctagtaaaag ataatgcaaa gagggaactc 3300

aaccatttac tagatgaaaa tattattacc tgttagttag actgaaaggc agtttgctag 3360
ctgatagaag gaggtacgaa ccaggtcagg tggcgattgt gttttttttt acgggtttgc 3420
ttagcggaat gaggaaacta cattcgacaa gagttactaa acatgatttg cagaaacact 3480
gtatggtatg gtattaatgt ttgtattata tttatagttc gtttgttcag ttctgttttt 3540
cgtttatttt aaaattactt tttttttggg acatacatat gtattttaat gatttcctta 3600
taattgagac tgagcatttg acaacacgaa ctttgcgtgt acggaagcaa agcgaaacaa 3660
aacaaagaaa caaacaaaaa acaagaaaat attgatggaa cccgctgact tttcgttgaa 3720
ctttatacaa agactgatta actaaaccgc aatttcggag ccaactaaag aaccatatat 3780
ggtgaggcca aaataagtga aagagcaaca cactcaaatt gcaatgacat tgataacgta 3840
aagtaactta acatagctta acatacatac tttcgcgcat atagagcgat acagatatca 3900
tatagatccg atcatccagt cacgaatcat catgggcata gctgaaaccg agtttaactg 3960
acattcatcg acatatttac ccatccatcc atccatccat ccactcactc ataccgatca 4020
tgcatcacag ggctttgttt ttcgtttgtc gttactttgt aattattgtg aaaggattta 4080
gccacaggat acagattcac ggcaggttga aaggaggagg aggaggaggg aggggttagc 4140
taaaattagt gggacatttt gggaagtaaa cgaaacagac ctgaaacaaa ataacgaaga 4200
agttataatt tttgactcaa tgtgttcatt agcatttagt agacttagcg ggcgtggcat 4260
agtgaaaacg tatggaaaat ccagagcaat ataagggcgt attgaataca catttaggtg 4320
acatgttcct cgcaaaagaa acaaataaaa caaaacaaaa gaaaaca 4367

<210> 11
<211> 263
<212> PRT
<213> human

<400> 11

Leu Cys Arg Pro Trp Gly Val Ser Val Asp Lys Glu Gly Tyr Ile Ile
1               5                   10                  15

Val Ala Asp Arg Ser Asn Asn Arg Ile Gln Val Phe Lys Pro Cys Gly
            20                  25                  30

Ala Phe His His Lys Phe Gly Thr Leu Gly Ser Arg Pro Gly Gln Phe
        35                  40                  45

Asp Arg Pro Ala Gly Val Ala Cys Asp Ala Ser Arg Arg Ile Val Val
    50                  55                  60

Ala Asp Lys Asp Asn His Arg Ile Gln Ile Phe Thr Phe Glu Gly Gln
65                  70                  75                  80

Phe Leu Leu Lys Phe Gly Glu Lys Gly Thr Lys Asn Gly Gln Phe Asn
                85                  90                  95

Tyr Pro Trp Asp Val Ala Val Asn Ser Glu Gly Lys Ile Leu Val Ser
            100                 105                 110

Asp Thr Arg Asn His Arg Ile Gln Leu Phe Gly Pro Asp Gly Val Phe
        115                 120                 125

Leu Asn Lys Tyr Gly Phe Glu Gly Ala Leu Trp Lys His Phe Asp Ser
    130                 135                 140

Pro Arg Gly Val Ala Phe Asn His Glu Gly His Leu Val Val Thr Asp
145                 150                 155                 160

Phe Asn Asn His Arg Leu Leu Val Ile His Pro Asp Cys Gln Ser Ala
                165                 170                 175

Arg Phe Leu Gly Ser Glu Gly Thr Gly Asn Gly Gln Phe Leu Arg Pro
            180                 185                 190

Gln Gly Val Ala Val Asp Gln Glu Gly Arg Ile Ile Val Ala Asp Ser
        195                 200                 205

Arg Asn His Arg Val Gln Met Phe Glu Ser Asn Gly Ser Phe Leu Cys
    210                 215                 220

Lys Phe Gly Ala Gln Gly Ser Gly Phe Gly Gln Met Asp Arg Pro Ser
225                 230                 235                 240

Gly Ile Ala Ile Thr Pro Asp Gly Met Ile Val Val Val Asp Phe Gly
                245                 250                 255

Asn Asn Arg Ile Leu Val Phe
            260

<210>  12
<211>  792
<212>  DNA
<213>  human

<400>  12
ctctgccgcc cttggggtgt gagtgtagac aaggagggct acatcattgt cgccgaccgc      60

agcaacaacc gcatccaggt gttcaagccc tgcggcgcct tccaccacaa attcggcacc     120

ctgggctccc ggcctgggca gttcgaccga ccagccggcg tggcctgtga cgcctcacgc     180

aggatcgtgg tggctgacaa ggacaatcat cgcatccaga tcttcacgtt cgagggccag     240

ttcctcctca agtttggtga gaaaggaacc aagaatgggc agttcaacta cccttgggat     300

gtggcggtga attctgaggg caagatcctg gtctcagaca cgaggaacca ccggatccag     360

ctgtttgggc ctgatggtgt cttcctaaac aagtatggct tcgagggggc tctctggaag     420

cactttgact ccccacgggg tgtggccttc aaccatgagg gccacttggt ggtcactgac     480

ttcaacaacc accggctcct ggttattcac cccgactgcc agtcggcacg ctttctgggc     540

tcggagggca caggcaatgg gcagttcctg cgcccacaag gggtagctgt ggaccaggaa 600

gggcgcatca ttgtggcgga ttccaggaac catcgggtac agatgtttga atccaacggc 660

agcttcctgt gcaagtttgg tgctcaaggc agcggctttg ggcagatgga ccgcccttcc 720

ggcatcgcca tcacccccga cggaatgatc gttgtggtgg actttggcaa caatcgaatc 780

ctcgtcttct aa 792

<210> 13
<211> 263
<212> PRT
<213> Mus musculus

<400> 13

Leu Cys Arg Pro Trp Gly Val Ser Val Asp Lys Glu Gly Phe Ile Ile
1               5                   10                  15

Val Ala Asp Arg Ser Asn Asn Arg Ile Gln Val Phe Lys Pro Cys Gly
            20                  25                  30

Ser Phe His His Lys Phe Gly Thr Leu Gly Ser Arg Pro Gly Gln Phe
        35                  40                  45

Asp Arg Pro Ala Gly Val Ala Cys Asp Ala Ser Arg Arg Ile Ile Val
    50                  55                  60

Ala Asp Lys Asp Asn His Arg Ile Gln Ile Phe Thr Phe Glu Gly Gln
65                  70                  75                  80

Phe Leu Leu Lys Phe Gly Glu Lys Gly Thr Lys Asn Gly Gln Phe Asn
                85                  90                  95

Tyr Pro Trp Asp Val Ala Val Asn Ser Glu Gly Lys Ile Leu Val Ser
            100                 105                 110

Asp Thr Arg Asn His Arg Ile Gln Leu Phe Gly Pro Asp Gly Val Phe
        115                 120                 125

Leu Asn Lys Tyr Gly Phe Glu Gly Ser Leu Trp Lys His Phe Asp Ser
    130                 135                 140

Pro Arg Gly Val Ala Phe Asn Asn Glu Gly His Leu Val Val Thr Asp
145                 150                 155                 160

Phe Asn Asn His Arg Leu Leu Val Ile His Pro Asp Cys Gln Ser Ala
                165                 170                 175

Arg Phe Leu Gly Ser Glu Gly Ser Gly Asn Gly Gln Phe Leu Arg Pro
            180                 185                 190

Gln Gly Val Ala Val Asp Gln Glu Gly Arg Ile Ile Val Ala Asp Ser

195                 200                 205

Arg Asn His Arg Val Gln Met Phe Glu Ala Asn Gly Ser Phe Leu Cys
    210                 215                 220

Lys Phe Gly Ala Gln Gly Ser Gly Phe Gly Gln Met Asp Arg Pro Ser
225                 230                 235                 240

Gly Ile Ala Val Thr Pro Asp Gly Leu Ile Val Val Val Asp Phe Gly
                245                 250                 255

Asn Asn Arg Ile Leu Ile Phe
            260

<210>  14
<211>  852
<212>  DNA
<213>  Mus musculus

<400>  14
tacgtgggca tcgggctccc cgggctgagc ttcggcagtg aaggtgacgg ggagggcaag      60

ctctgcaggc cctggggcgt gagtgttgat aaggagggtt tcatcatagt ggctgaccga     120

agcaataaca gaatccaggt gtttaagccc tgtggttcct tccaccacaa gtttggtacc     180

cttgggtctc gtcctgggca gtttgaccga ccggctgggg tggcttgtga cgcctcccgc     240

aggatcatag tggccgacaa agacaatcat cgcattcaga tcttcacctt cgagggccag     300

ttccttctta agtttggtga gaaaggcacc aagaacgggc agtttaacta cccgtgggat     360

gtagcagtga attctgaggg caagatcctc gtttcagaca cccggaatca tcgcatccag     420

ttgttcggac cggacggggt cttcctgaac aagtacggct tcgagggatc tctctggaag     480

cacttcgact ctccacgggg cgtggctttc aacaatgaag gtcacctggt agtcaccgac     540

ttcaacaacc accggcttct ggttattcac cctgattgcc aatctgcccg cttcttgggt     600

tcggagggct cgggcaatgg gcagttcctg cgaccacagg gtgtggctgt ggaccaggaa     660

gggagaatca tcgtagccga ttccagaaat caccgcgtgc agatgtttga ggccaatggc     720

agcttcctgt gcaagtttgg tgctcaaggg agtggcttcg ggcagatgga ccgcccctca     780

ggtatcgcgg tcaccccgga tggattgatc gtcgtggtgg actttggcaa caatcgaatc     840

ctcatcttct aa                                                         852

<210>  15
<211>  283
<212>  PRT
<213>  Drosophila melanogaster

<400>  15

Val Ser Arg Pro Trp Gly Leu Cys Val Asp Lys Met Gly His Val Leu
1               5                   10                  15

Val Ser Asp Arg Arg Asn Asn Arg Val Gln Val Phe Asn Pro Asp Gly

20                  25                  30

Ser Leu Lys Phe Lys Phe Gly Arg Lys Gly Val Gly Asn Gly Glu Phe
        35                  40                  45

Asp Leu Pro Ala Gly Ile Cys Val Asp Val Asp Asn Arg Ile Ile Val
    50                  55                  60

Val Asp Lys Asp Asn His Arg Val Gln Ile Phe Thr Ala Ser Gly Val
65                  70                  75                  80

Phe Leu Leu Lys Phe Gly Ser Tyr Gly Lys Glu Tyr Gly Gln Phe Gln
                85                  90                  95

Tyr Pro Trp Asp Val Ala Val Asn Ser Arg Arg Gln Ile Val Val Thr
            100                 105                 110

Asp Ser Arg Asn His Arg Ile Gln Gln Phe Asp Ser Glu Gly Arg Phe
        115                 120                 125

Ile Arg Gln Ile Val Phe Asp Asn His Gly Gln Thr Lys Gly Ile Ala
    130                 135                 140

Ser Pro Arg Gly Val Cys Tyr Thr Pro Thr Gly Asn Ile Ile Val Ser
145                 150                 155                 160

Asp Phe Asp Asn His Cys Leu Tyr Leu Ile Asp Pro Asp Ile Asn Asp
                165                 170                 175

Ile Leu Ser Val Lys Gly His Glu Gly Ser Gly Phe His Glu Phe Asn
            180                 185                 190

Arg Pro Ser Gly Leu Cys Cys Asp Asp Glu Gly Arg Ile Ile Val Ala
        195                 200                 205

Asp Ser Lys Asn Gln Arg Ile Leu Val Phe Asn Gln Asn Leu Asp Phe
    210                 215                 220

Met Trp Asp Ile Glu Val Arg Pro Ser Ile Asn Pro Leu Met Pro Pro
225                 230                 235                 240

Thr Leu Asp Glu Lys Asp Arg Thr Cys Asp Val Ala Ile Met Pro Asp
                245                 250                 255

Gly Arg Ile Val Phe Leu Ile Glu Leu Ser Pro Asp Ser Lys Glu Gly
            260                 265                 270

Ser Asn Pro Tyr Lys Arg Phe Val His Val Phe
        275                 280

<210>  16

<211>  1293
<212>  DNA
<213>  Drosophila melanogaster

<400>  16
gtgagccggc catggggtct atgcgttgat aaaatgggcc acgttctcgt atcggatcgc      60

cgcaacaatc gcgtccaggt cttcaatccc gatggctccc taaagttcaa attcggccgt     120

aaaggcgtgg gcaatggaga gttcgatctg cctgctggaa tctgtgttga cgttgataat     180

cgcattattg tggtcgacaa ggacaatcac cgtgtgcaaa tctttaccgc cagcggcgtc     240

ttcctgctca agttcggcag ctacggcaag gagtacggcc agttccagta tccgtgggat     300

gtggccgtga actcgcgccg ccaaattgtg gttaccgact cgcgcaatca tcgcatccag     360

cagttcgact ctgagggtcg cttcatccgc cagatagtgt tcgacaacca tgggcaaacc     420

aaaggaatcg cctcgccacg aggcgtttgc tacacgccga cgggcaacat catagtgtct     480

gactttgaca accactgcct ctacctgatt gatccagaca tcaacgacat tctttccgtg     540

aagggacatg agggctcggg cttccatgaa tttaatcgac cctccggcct gtgctgtgac     600

gacgagggcc gcattatagt ggccgattca aagaatcaac gcatcctggt cttcaatcag     660

aacctggact ttatgtggga tatcgaggtg cgaccctcca tcaatccctt gatgccgccc     720

acgctggacg agaaagatcg cacttgcgat gtggccataa tgccagacgg tcgcattgtg     780

ttcctcattg aactgtcgcc agactccaag gaagggtcaa acccttacaa gcggtttgtg     840

cacgtattct aaataaggcc acgagtgccg atcttttttt ttagaccatt tacaagagcg     900

aaaccttagc gtagagtttc aaataacatg acgattatga ggaggtgccg aaaccgattt     960

aataagaaaa aatctgaaat catgaaagtt agcgaaaatt tttagcttaa gtgctacaac    1020

gattacctcg atattgctta agagttatgg ttacgatttg gtaatttaca atacaatacg    1080

aattacacat atgtatctat ggatatctat atctatacgc atatgcatat atctagcgaa    1140

tatacaagcc caaccaacta gaactataac taaaacaaaa acaaaaaaca aaaactaaag    1200

cgaaaggcag gcggttgagt aggatttagg agatatatat aaacgtaggg tccgtgagca    1260

ttcagtagag ttaccgaacc tagtaaaaga taa                                 1293


<210>  17
<211>  16
<212>  PRT
<213>  Mus musculus

<400>  17

Lys Phe Gly Glu Lys Gly Thr Lys Asn Gly Gln Phe Asn Tyr Pro Trp
1               5                   10                  15


<210>  18
<211>  16
<212>  PRT
<213>  Mus musculus

<400>  18

Cys Val Arg Ala His Gln Arg Val Arg Leu Thr Lys Asp His Tyr Ile
1               5                   10                  15

<210>  19
<211>  15
<212>  PRT
<213>  Drosophila melanogaster

<400>  19

Leu Ser Leu Ser Phe Ala Thr Glu Gly His Glu Asp Gly Gln Val
1               5                   10                  15

<210>  20
<211>  18
<212>  PRT
<213>  Drosophila melanogaster

<400>  20

Ser Pro Asp Ser Lys Glu Gly Ser Asn Pro Tyr Lys Arg Phe Val His
1               5                   10                  15

Val Phe